# EUROPÄISCHE PATENTSCHRIFT

(11) **EP 1 957 103 B1**
(45) Veröffentlichungstag und Bekanntmachung des Hinweises auf die Patenterteilung: **20.08.2014**
(21) Anmeldenummer: 06818865.5
(22) Anmeldetag: 21.11.2006
(51) Int. Cl.: A61K 39/02, A61K 39/04, A61K 39/05, C07K 14/30, A61K 39/112

(54) **BAKTERIELLER IMPFSTOFF GEGEN BAKTERIELLE INFEKTIONSERREGER FÜR DIE ANWENDUNG BEI TIEREN**
BACTERIAL VACCINE AGAINST BACTERIAL INFECTIVE AGENTS, TO BE USED IN ANIMALS
VACCIN BACTERIEN POUR LUTTER CONTRE DES AGENTS INFECTIEUX BACTERIENS, EMPLOYE CHEZ DES ANIMAUX

(30) Priorität: 30.11.2005 DE 102005057643
(43) Veröffentlichungstag der Anmeldung: 20.08.2008
(73) Patentinhaber: Stiftung Tierärztliche Hochschule Hannover, 30173 Hannover (DE)
(72) Erfinder: GERLACH, Gerald-F., 30449 Hannover (DE); MEENS, Jochen, 30952 Ronnenberg (DE); MAAS, Alexander, 59590 Geseke (DE); SELKE, Martin, 30459 Hannover (DE)
(74) Vertreter: Gross, Felix
(86) Internationale Anmeldenummer: PCT/EP2006/011380
(87) Internationale Veröffentlichungsnummer: WO 2007/062795

(56) Entgegenhaltungen:
- KOTLOFF K L ET AL: "Shigella flexneri 2a strain CVD 1207, with specific deletions in virG, sen, set, and guaBA, is highly attenuated in humans." INFECTION AND IMMUNITY MAR 2000, Bd. 68, Nr. 3, März 2000 (2000-03), Seiten 1034-1039, XP002454965 ISSN: 0019-9567
- MASTROENI P ET AL: "Salmonella: immune responses and vaccines." VETERINARY JOURNAL (LONDON, ENGLAND : 1997) MAR 2001, Bd. 161, Nr. 2, März 2001 (2001-03), Seiten 132-164, XP002454966 ISSN: 1090-0233
- TACKET C O ET AL: "Safety of live oral Salmonella typhi vaccine strains with deletions in htrA and aroC aroD and immune response in humans." INFECTION AND IMMUNITY FEB 1997, Bd. 65, Nr. 2, Februar 1997 (1997-02), Seiten 452-456, XP002454967 ISSN: 0019-9567
- SELKE-M: "Changing the strategy: OmpD as marker in a negative-marker vaccine against Salmonella Typhimurium?" 2005, CONFERENCE OF RESEARCH WORKERS IN ANIMAL DISEASES: PROCEEDINGS OF THE 86TH ANNUAL MEETING, ST. LOUIS, MISSOURI, 04.-06.12.2005; AMES, IA, USA: BLACKWELL, 2005, S. 95 ISBN 0-8138-2680-2 , PUBLICATION DATE 2.1.2006 , XP001249362 das ganze Dokument
- MEYER P N ET AL: "Virulence of a Salmonella typhimurium OmpD mutant." INFECTION AND IMMUNITY JAN 1998, Bd. 66, Nr. 1, Januar 1998 (1998-01), Seiten 387-390, XP002455109 ISSN: 0019-9567
- DORMAN C J ET AL: "CHARACTERIZATION OF PORIN AND OMP-R MUTANTS OF A VIRULENT STRAI OF SALMONELLA -TYPHIMURIUM OMP-R MUTANTS ARE ATTENUATE IN-VIVO" INFECTION AND IMMUNITY, AMERICAN SOCIETY FOR MICROBIOLOGY. WASHINGTON, US, Bd. 57, Nr. 7, Juli 1989 (1989-07), Seiten 2136-2140, XP002112175 ISSN: 0019-9567
- HENDERSON ET AL: "Overview of marker vaccine and differential diagnostic test technology" BIOLOGICALS, ACADEMIC PRESS LTD., LONDON, GB, Bd. 33, Nr. 4, 28. Oktober 2005 (2005-10-28), - Dezember 2005 (2005-12) Seiten 203-209, XP005176932 ISSN: 1045-1056
- SHAMS ET AL: "Recent developments in veterinary vaccinology" VETERINARY JOURNAL, BAILLIERE TINDALL, LONDON,, GB, Bd. 170, Nr. 3, 3. Mai 2005 (2005-05-03), - November 2005 (2005-11) Seiten 289-299, XP005146204 ISSN: 1090-0233
- SANTIVIAGO CARLOS A ET AL: "Global regulation of the Salmonella enterica serovar typhimurium major porin, OmpD." JOURNAL OF BACTERIOLOGY OCT 2003, Bd. 185, Nr. 19, Oktober 2003 (2003-10), Seiten 5901-5905, XP002454968 ISSN: 0021-9193
- CURTISS ROY 3RD: "Bacterial infectious disease control by vaccine development." THE JOURNAL OF CLINICAL INVESTIGATION OCT 2002, Bd. 110, Nr. 8, Oktober 2002 (2002-10), Seiten 1061-1066, XP002454969 ISSN: 0021-9738
- VAN OIRSCHOT J T: "Present and future of veterinary viral vaccinology: A review" VETERINARY QUARTERLY, KLUWER, DORDRECHT, NL, Bd. 23, Nr. 3, Juli 2001 (2001-07), Seiten 100-108, XP002964190 ISSN: 0165-2176
- ROTH J A ET AL: "New technology for improved vaccine safety and efficacy." THE VETERINARY CLINICS OF NORTH AMERICA. FOOD ANIMAL PRACTICE NOV 2001, Bd. 17, Nr. 3, November 2001 (2001-11), Seiten 585-597 , vii, XP009091017 ISSN: 0749-0720
- KOIDE Y ET AL: "DNA vaccines." JAPANESE JOURNAL OF PHARMACOLOGY JUL 2000, Bd. 83, Nr. 3, Juli 2000 (2000-07), Seiten 167-174, XP002455149 ISSN: 0021-5198

## Beschreibung

Die Erfindung betrifft einen bakteriellen Impfstoff nach dem Oberbegriff des Anspruchs 1 und ein Verfahren zu dessen Herstellung nach Anspruch 3. Des Weiteren betrifft die Erfindung ein Bakterium nach Anspruch 4, ein immunogenes Protein nach Anspruch 7, dessen Verwendung nach Anspruch 10 und ein diagnostisches Verfahren nach Anspruch 11.

In der landwirtschaftlichen Nutztierhaltung, insbesondere in der Schweinehaltung, sind Atemwegserkrankungen und Infektionen des Magen-Darm-Traktes für erhebliche wirtschaftliche Schäden verantwortlich. Die Erkrankungen verursachen nicht nur eine hohe Mortalität, verringerte Wachstumsraten und schlechtere Fleischqualität, sondern sind auch für mehr als die Hälfte aller antibiotischen Behandlungen im Bereich der Mastschweinehaltung verantwortlich (Elbers et al., 1990, Sero-epidemiological screening of pig sera collected at the slaughterhouse to detect herds infected with Aujeszky's disease virus, porcine influenza virus and Actinobacillus (Haemophilus) pleuropneumoniae in the framework of an integrated quality control system. Vet.Q. 12, 221-230).

Impfungen, insbesondere gegen bakterielle Infektionen, werden in der landwirtschaftlichen Nutztierhaltung fast ausschließlich metaphylaktisch und nicht prophylaktisch eingesetzt. Im Zentrum der Maßnahmen steht dabei der Bestand und nicht das Einzeltier. So werden insbesondere Bestände, in denen ein Erreger endemisch ist, gegen diesen Erreger geimpft, unabhängig davon, ob ein Einzeltier bereits infiziert ist oder nicht.

Die gegenwärtig in der Tiermedizin verwendeten Impfstoffe gegen bakterielle Infektionserreger sind ganz überwiegend einfache mit Adjuvans formulierte Bakterine z.B. Formalin-inaktivierte ganze Bakterien. Unter einem Adjuvans ist hierbei eine Substanz zu verstehen, die als Mischung mit einer Substanz (Antigen), die eine Immunantwort auslöst und die Antikörper induziert, oder getrennt davon appliziert wird und in unspezifischer Weise die Immunantwort auf dieses Antigen verstärkt oder verändert. Sie bewirken einen häufig serotypischen Schutz vor klinischen Erkrankungen ohne jedoch eine latente Besiedlung der Schleimhäute zu verhindern. Dadurch kann der Infektionserreger auch durch geimpfte Tiere weiterverbreitet werden.

Die bedeutsamsten Erreger von Ateminfektionskrankheiten wie *Actinobacillus pleuropneumoniae, Mycoplasma hyopneumoniae* oder *Mycobacterium bovis* sind hoch kontagiös und treten häufig als latente Besiedler von Schleimhäuten des Atemtraktes auf. Insbesondere A. *pleuropneumoniae* und *M. hyopneumoniae* ist gemeinsam, dass rekonvaleszente Tiere häufig über Monate Träger des Erregers bleiben. Dadurch ist es, unterstützt durch Viehhandel und Tiertransporte, zu einer flächendeckenden Verbreitung der Erreger gekommen. Die bisher auf dem Markt befindlichen Impfstoffe sind fast ausschließlich Bakterine. Keiner der Impfstoffe verhindert eine latente Infektion oder erlaubt eine Unterscheidung zwischen geimpften und infizierten Tieren.

Infektionen mit *Salmonella* Typhimurium stellen - unter dem Gesichtspunkt des Gesundheitlichen Verbraucherschutzes - die wichtigste Magen-Darm-Trakt-Erkrankung bei Schweinen dar. Impfstoffe sind bisher gegen die Salmonellose der Kälber und gegen die Salmonellenbesiedlung der Schweine und der Legehennen auf dem Markt. In allen Fällen sind auch oder ausschließlich attenuierte Lebendimpfstoffe erhältlich. Bei Kälbern wird durch die Impfung das Auftreten klinischer Erscheinungen wirkungsvoll verhindert. Bei Legehennen werden die intraovarielle Übertragung sowie die Ausscheidung des Erregers deutlich reduziert, bei Schweinen wird die Ausscheidung des Erregers reduziert.

Die zugelassenen Salmonellen-Lebendimpfstoffe beinhalten durch chemische Mutationen hergestellte attenuierte Erreger, die genotypisch und phänotypisch vom Wildstamm unterschieden werden können. Eine serologische Differenzierung geimpfter und infizierter Tiere ist aber bei Anwendung dieser Impfstoffe nicht möglich.

Die gegenwärtigen Bakterinimpfstoffe erlauben somit keine serologische Unterscheidung von geimpften und infizierten Nutztieren z.B. durch Anwendung eines ELISA-Tests. Damit ist es nicht möglich, mit Hilfe dieser Impfstoffe spezifiziert pathogenfreie (SPF) Bestände aufzubauen und unter Impfschutz kontrollierbar zu halten. Die Erzeugung von SPF-Beständen ist im Sinne des Vorbeugenden Verbraucherschutzes und der Wirtschaftlichkeit jedoch das Idealziel.

Weiterhin ist es problematisch, Schweine in Salmonellen-kontrollierten Betrieben (BMVEL 1996; Leitlinie zur Reduzierung des Eintrags von Salmonellen durch Schlachtschweine in die Fleischgewinnung; eine entsprechende Verordnung ist in Vorbereitung) zu impfen, da die Einstufung und Überwachung dieser Betriebe über die Serologie erfolgt und geimpfte Tiere serologisch positiv reagieren.

Das Ziel der Erzeugung von SPF-Beständen wurde für die Aujeszky'sche Krankheit bei Schweinen (verursacht durch das Porcine Herepsvirus I) und bei Infektionen von Rindern mit dem Bovinen Herpesvirus 1 (BHV1) durch die Einführung von Marker-Impstoffen erreicht. Markerimpfstoffe basieren auf strukturell veränderten Impfstämmen die eine i.d.R. serologische Unterscheidung von vakzinierten und natürlich infizierten Tieren ermöglichen. Man unterscheidet zwischen Positiv- und Negativmarker-Impstoffen.

Zur Herstellung von Positiv-Markerimpfstoffen wird in der Regel ein Marker-Antigen gentechnisch in den Impfstamm eingeführt, das i.d.R. die Bildung zusätzlicher Antikörper induziert oder zumindest genotypisch oder phänotypisch nachweisbar ist. Nachteilig ist, dass spätere Infektionen mit einem nativen Infektionserreger nach einer Impfung, d.h. geimpft plus infiziert, nicht mehr nachgewiesen werden können. Der Positivmarker-Impfstoff erlaubt also nur die Unterscheidung von ungeimpften, ungeimpften plus infizierten und geimpften Tieren.

Negativ-Markerimpfstoffe sind dadurch gekennzeichnet, dass dem Genom des Erregers ein definierter Bereich des Genoms fehlt. Diese Mutationen können auf natürlichem oder gentechnischem Wege entstehen. Das Fehlen eines definierten Abschnittes aus einem für ein immunogenes Protein kodierenden Gen führt zur Veränderung der humoralen Immunantwort, die eine Unterscheidung zwischen nur infizierten, nur geimpften und geimpften plus infizierten Tiere erlaubt. Eine Abgrenzung von geimpften plus infizierten und nur infizierten Tieren ist nicht möglich, wobei diese Abgrenzung jedoch medizinisch und wirtschaftlich irrelevant ist. Der Vorteil von Negativmarker-Impfstoffen ohne Fremd-DNA ist zudem, dass es sich zwar um gentechnisch veränderte Organismen handelt, die aber nicht unter das deutsche Gentechnikgesetz fallen.

Zur Vorbeugung und Bekämpfung bakterieller Infektionskrankheiten bei Nutztieren sind jedoch bis heute, anders als im Bereich der viralen Infektionskrankheiten, gezielt keine Markerimpfstoffe entwickelt worden.

KOTLOFF K L ET AL: "Shigella flexneri 2a strain CVD 1207, with specific deletions in virG, sen, set, and guaBA, is highly attenuated in humans." INFECTION AND IMMUNITY MAR 2000, Bd. 68, Nr. 3, März 2000 (2000-03), Seiten 1034-1039, XP002454965 ISSN: 0019-9567 beschreibt ein Vakzin aus Shigella flexneri 2a Stamm CVD 1207 (Enterobacteriaceae) mit spezifischen Delefionsmutationen in den Genen virG, sen, set und guaBA.

Der Übersichtsartikel MASTROENI P ET AL: "Salmonella: immune responses and vaccines." VETERINARY JOURNAL (LONDON, ENGLAND : 1997) MAR 2001, Bd. 161, Nr. 2, März 2001 (200103), Seiten 132-164, XP002454966 ISSN: 1090-0233 beschreibt die Bereitstellung von Impfstoffen gegen Salmonella Infektionen. Als geeignete Impfstoffe werden verschiedene Ein- bis Dreifachmutanten und beschrieben. In einer Tabelle sind diverse Gentargets aufgeführt.

MEYER P N ET AL: "Virulence of a Salmonella typhimurium OmpD mutant." INFECTION AND IMMUNITY JAN 1998, Bd. 66, Nr. 1, Januar 1998 (1998-01), Seiten 387-390, XP002455109 ISSN: 0019-9567 beschreibt *Salmonella* Typhimurium OmpD Mutanten. Diese Mutanten sind jedoch nicht als bakterieller Impfstoff geeignet, da sich diese in ihrer Virulität nicht vom Wildtyp unterscheiden.

DORMAN C J ET AL: "CHARACTERIZATION OF PORIN AND OMP-R MUTANTS OF A VIRULENT STRAI OF SALMONELLA -TYPHIMURIUM OMP-R MUTANTS ARE ATTENUATE IN-VIVO" INFECTION AND IMMUNITY, AMERICAN SOCIETY FOR MICROBIOLOGY. WASHINGTON, US, Bd. 57, Nr. 7, Juli 1989 (1989-07), Seiten 2136-2140, XP002112175 ISSN: 0019-9567 betrifft *Salmonella* Typhimirium Mutanten mit Deletionen in den Porinen ompC, ompD, ompF und dem Regulator ompR, wobei die Mutation in ompR zu einem avirulenten Stamm führt, während die anderen Mutationen die Virulenz gar nicht oder nur geringfügig beeinflusst.

Der vorliegenden Erfindung liegt daher die Aufgabe zugrunde, Markerimpfstoffe zur Bekämpfung bakterieller Infektionskrankheiten zu entwickeln, die eine einfache serologische Unterscheidung von geimpften und natürlich infizierten landwirtschaftlichen Tieren, insbesondere in der Schweinehaltung, ermöglichen.

Diese Aufgabe wird durch einen bakteriellen Impfstoff mit den Merkmalen des Anspruchs 1 gelöst.

Der erfindungsgemäße bakterielle Impfstoff für die Anwendung bei Tieren, insbesondere bei Nutztieren, umfasst mindestens einen Serotyp des Organismus *Salmonella enterica* mit mindestens jeweils einer Knock-out Mutationen in mindestens drei verschiedenen chromosomalen Genen, wobei der Serotyp *Salmonella* Thypimurium mit mindestens einer Knock-out Mutation in einem Gen kodierend für das Protein OmpD umfasst.

Die knock-out Mutation in dem für das immunogene Protein kodierenden Bereich bewirkt somit, dass das korrespondierende immunogene Protein und somit auch die entsprechenden Antikörper in einem geimpften Tier nicht mehr gebildet werden. Da das durch die Genmanipulation nicht länger exprimierte immunogene Protein jedoch für eine protektive Immunität nicht essentiell ist, bietet der erfindungsgemäße Impfstoff nach wie vor einen effektiven Immunschutz.

Die für die protektive Immunantwort essentiellen Proteine werden hingegen nicht manipuliert, so dass der Impfstoff einen aktiven Schutz des geimpften Tieres gegen den jeweiligen Infektionserreger bietet. Somit ist es möglich, zwischen geimpften und natürlich infizierten Tieren durch einfache serologische Tests wie ELISA zu unterscheiden, da der natürliche, nicht manipulierte Erreger alle immunogenen Proteine produziert, dem als Impfstoff verwendete Erreger jedoch einige für die Protektion nicht erforderliche immunogene Proteine fehlen.

Die Manipulierung des Infektionserregers basiert somit im Wesentlichen auf für die Protektion nicht essentiellen immunogenen Proteinen und deren korrespondierenden Genen, auf einem genetisch manipulierbaren Targetstamm und einem System zur Gegenselektion zur Identifizierung des manipulierten Targetstammes.

Bevorzugt sind weitere Knock-out Mutationen in chromosomale Gene eingeführt, die nicht für immunogene Proteine kodieren, die jedoch der Attenuierung des bakteriellen Erregers dienen.

Der Impfstoff umfasst mindestens einen Infektionserreger aus der Gruppe der gram-negativen Bakterien, insbesondere aus der Familie der Enterobakterien, *Pasteurellaceae* oder Mycoplasmen, die verantwortlich sind für verschiedene Infektionskrankheiten bei Nutztieren.

Der im Impfstoff enthaltende Infektionserreger kann auch mindestens ein Serotyp von *Actinobacillus pleuropneumoniae* oder *Mycoplasma hyopneumoniae* sein.

Der Impfstoff weist auch vorteilhafterweise mindestens einen Infektionserreger aus der Gruppe der gram-positiven Bakterien auf, bevorzugt aus der Familie der Atemwegserkrankungen verursachenden Mykobakterien wie *Mycobacterium bovis.*

Die in den Infektionserreger eingeführten Knock-out Mutationen stellen vorteilhafterweise unmarkierte Deletionen dar, wobei unter einer unmarkierten Deletion eine Deletion eines Genomabschnittes ohne Einführung eines speziellen Selektionsmarkers wie Antiobiotika-Resistenz-Kassetten in diesen Genomabschnitt zu verstehen ist.

Mit Vorteil weist mindestens ein Serotyp des Organismus *Actinobacillus pleuropneumoniae* Knock-out Mutationen in mindestens drei der Gene auf, die für die Proteine ApxIIA (Acc.No. AAU847009), UreC (Acc. No. AAC00060), DmsA (Acc. No. AAN28298), AspA (Acc. No. NC_003998) HypB (Acc. No. ZP_00134397) und / oder FurA (NC_004130) kodieren.

Das Protein ApxIIA ist ein sezerniertes, stark immunogenes Toxin, das bei allen Serotypen von A. *pleuropneumoniae* mit Ausnahme vom Serotyp 10 vorkommt. UreC ist eine Komponente des Ureaseenzymkomplexes, der Harnstoff zu Ammoniak und Kohlendioxid hydrolysiert und damit dem Erreger die Persistenz im sauren Milieu der "epithelial lining fluid" der Lunge erleichtert.

Das dmsA-Gen kodiert für eine unter anaeroben Bedingungen exprimierte, Virulenzassoziierte DMSO-Reduktase (Baltes et al., 2003, Identification of dimethyl sulfoxide reductase in Actinobacillus pleuropneumoniae and its role in infection. Infect.Immun. 71, 6784-6792). Eine entsprechende Einzelmutante von *A. pleuropneumoniae* Serotyp 7 erwies sich im Infektionsversuch als leicht attenuniert.

Das *aspA* Gen kodiert für eine Untereinheit der Aspartat-Ammonium-Lyase (Jacobsen et al. 2005, Enzymes involved in anaerobic respiration appear to play a role in Actinobacillus pleuropneumoniae virulence. Infect Immun 73, 226-234) und das *hybB* Gen kodiert für das Enzym [Ni,Fe]-Dehydrogenase 2 (Baltes, Kyaw et al., 2004, Lack of influence of the anaerobic [NiFe] hydrogenase and L-1,2 propanediol oxidoreductase an the outcome of Actinobacillus pleuropneumoniae serotype 7 infection. Vet.Microbiol 102, 67-72). Beide Enzyme haben eine Bedeutung im anaeroben Stoffwechsel von A. *pleuropneumoniae* und ermöglichen es dem Erreger, im anaeroben Milieu der Lungenläsionen zu überleben.

Das *furA* Gen (ferric uptake regulation, *fur*) kodiert für einen globalen eisenabhängigen Regulator. Dieser ist ein transkriptionaler Repressor von eisenregulierten Promotoren, der an die DNA bindet, wenn Eisen vorhanden ist. Eine *fur*-Mutante von A. *pleuropneumoniae* Serotyp 7 wurde bereits konstruiert und im Tier geprüft (Jacobsen et al. 2005, Enzymes involved in anaerobic respiration appear to play a role in Actinobacillus pleuropneumoniae virulence. Infect Immun 73, 226-234). Dabei wurde festegestellt, dass die Mutante nur noch sehr geringfügige Krankheitssymptome verursacht, aber noch eine starke Immunantwort induziert.

Die Aufgabe der Erfindung wird ebenfalls durch ein Verfahren zur Herstellung des Impfstoffes nach Anspruch 3 gelöst.

Das erfindungsgemäße Verfahren zu Herstellung des Impfstoffes ist dadurch charakterisiert, dass zur Mutagenese von OmpD im Serotyp *Salmonella* Thypimurium ein pROKB1 Vektor mit einem R6K Replikon verwendet wird, welcher das OmpD Gen mit einer Knock-out Mutation enthält. Das Transkonjugationsplasmid enthält ein Gen des Infektionserregers mit mindestens einer Kock-out Mutation. Bevozugt enthält das Gen eine interne Deletion und kodiert für ein immunogenes Protein, das für den effektiven Immunschutz nicht essentielle Antikörper generiert.

Das Transkonjugationsplasmid beschrieben in der DE 19928073 umfasst die Mobilisierungsregion mobRP4, einen Polylinker, ein Replikon des ColE1 Typs, eine Resistenzdeterminante und eine transkriptionelle Fusion des *Bacillus subtilis* sacB-Gens mit dem *Actinobacillus pleuropneumoniae* omIA-Promoter. Das für ein immunogenes Protein kodierende Gen mit einer unmarkierten Deletion ist dabei als exogene DNA in den Polylinker insertiert.

Die Einführung der Knock-out Mutationen in die mindestens drei chromosomale Gene erfolgt vorteilhafterweise nacheinander.

Der erfindungsgemäße Impfstoff wird vorteilhafterweise als bakterieller Lebend-Negativmarker Impfstoff und bakterieller Inaktivat-Negativ-Marker-Impfstoff verwendet.

Ein Lebend-Impfstoff umfasst dabei einen attenuierten, infektiösen aber abgeschwächten Stamm, der replikationsfähig aber avirulent ist. Im Gegensatz dazu ist ein Inaktivat-Impfstoff nicht replikationsfähig. Die Inaktivierung erfolgt durch Alkohole oder Aldehyde. Die Induktion einer Immunantwort wird durch Adjuvantien wie Al(OH)₃ ausgelöst. Bei Subunitimpfstoffen ist keine Inaktivierung erforderlich.

Die Lösung der erfindungsgemäßen Aufgabe wird auch durch ein Bakterium nach Anspruch 4 verwendet im erfindungsgemäßen bakteriellen Impfstoff sowie einem diagnostischen Verfahren nach Anspruch 11 erreicht.

Das diagnostisches Verfahren zur Unterscheidung von infizierten Tieren und mit dem erfindungsgemäßen bakteriellen Impfstoff geimpften Tieren ist durch die serologische Bestimmung von für die Immunprotektion nicht essentiellen immunogenen Proteinen, insbesondere mittels eines ELISA-Tests, gekennzeichnet.

Die gestellte Aufgabe wird auch durch ein immunogenes Protein nach Anspruch 7 und deren Verwendung nach Anspruch 10 gelöst.

Demnach ist das erfindungsgemäße immunogene Protein von mindestens einem bakteriellen Infektionserreger eines Tieren verwendet in einem Impfstoff dadurch charakterisiert, dass es Antikörper generiert, die bevorzugt für eine protektive Immunantwort nicht essentiell sind, wobei es mit Antikörpern aus mindestens einem Serum von mit *Salmonella* Typhimurium infizierten Schweinen und mit Antikörpern aus mindestens einem Serum von gegen *Salmonella* Typhimurium geimpften Schweinen wechselwirkt.

Das erfindungsgemäße Proteins ist bevorzugt dadurch nachweisbar, dass das Protein mit Antikörpern aus mindestens einem Serum von einem natürlich infizierten oder einem natürlich infizierten und geimpften Tieren wechselwirkt, aber nicht oder nur teilweise mit Antikörpern aus mindestens einem Serum von nur mit Markerimpfstoffen geimpften Tieren derselben Spezies wechselwirkt.

Die spezifische Wechselwirkung des erfindungsgemäßen Proteins mit den Seren eines natürlich infizierten oder natürlich infizierten plus geimpften Tieres basiert auf der Bindung eines in den Seren vorhandenen Antikörpers an sein spezifisches Antigen.

Bei einer Infektion eines Tieres mit einem natürlichen Infektionserreger werden normalerweise eine Vielzahl von immunogenen Proteinen des Infektionserregers exprimiert, sowohl für den Immunschutz essentielle Proteine als auch nicht-essentielle Proteine. Die als Lebendimpfstoffe verwendeten Infektionserreger sind hingegen in ihrer Virulenz abgeschwächte Bakterien, die so konstruiert sind, dass sie mindestens ein immunogenes Protein, das nicht für die Immunisierung essentiell ist, nicht exprimieren. Die Seren von geimpften Tieren enthalten also ein anderes Spektrum an Antikörpern und unterscheiden sich somit von den Seren der natürlich infizierten Tiere.

Die erfindungsgemäßen Proteine sind somit bevorzugt in natürlich infizierten oder in natürlich infizierten plus geimpften Tieren detektierbar und bilden daher die Grundlage für die serologischen Testverfahren zur Unterscheidung von mit dem erfindungsgemäßen Impfstoff geimpften Tieren und natürlich infizierten sowie natürlich infizierten plus geimpften Tieren.

Mit Vorteil zeigt das immunogene Protein eine Wechselwirkung mit Antikörpern aus Seren von mit *Mycoplasma hyopneumoniae* infizierten Schweinen, jedoch keine oder nur eine schwache Wechselwirkung mit Antikörpern aus Seren von gegen *Mycoplasma hyopneumoniae* geimpften Schweinen. Der bei dieser Impfung verwendete Impfstoff basiert vorteilhafterweise auf abgetöteten *Mycoplasma hyopneumoniae* Erregern. Bevorzugte immunogene Proteine sind die hypothetischen Lipoproteine Protein MHP 378 (Acc. No. YP_115889) und MHP 651 (Acc. No. YP_116159) aus *Mycoplasma hyopneumoniae.*

Das erfindungsgemäße immunogene Protein ist aber auch dadurch nachweisbar, dass es bevorzugt mit Antikörpern aus mindestens einem Serum von mit *Salmonella* Typhimurium infizierten Schweinen und mit Antikörpern aus mindestens einem Serum von gegen *Salmonella* Typhimurium geimpften Schweinen wechselwirkt. Ein bevorzugtes immunogenes Protein identifiziert in *Salmonella* Typhimurium ist das Protein OmpD.

Um einen bereits vorhandenen Lebendimpfstoff gegen *Salmonella* Typhimurium mit einem Marker zu versehen, ist es notwendig, dass das zu mutierende Gen für ein immunogenes Protein kodiert, das von Antikörpern erkannt wird, die sowohl von natürlich infizierten als auch in geimpften Tieren generiert werden. Seren aus Tieren, die den mit dem entsprechenden Marker in Form eines deletierten Genes versehenen Lebendimpfstoff erhalten, reagieren mit diesem Protein später nicht mehr.

Das erfindungsgemäße Protein, und deren korrespondierende Nukleinsäure bildet die Grundlage zur Mutagenese des jeweiligen Infektionserregers zur Entwicklung eines Marker-Impfstoffes.

Bevorzugt wird das erfindungsgemäße Protein zur Detektion von natürlich infizierten Tieren und / oder natürlich infizierten und geimpften Tieren, insbesondere von Schweinen, und / oder als Impfstoff verwendet.

Des Weiteren wird die gestellte Aufgabe durch eine Nukleinsäure und deren Verwendung gelöst.

Die Nukleinsäure kodiert für mindestens ein immunogenes Protein, das bevorzugt für eine protektive Immunantwort nicht essentielle Antikörper induziert, und ist durch mindestens eine Mutation zur Anpassung an die spezifische Kodonnutzung von mindestens einem geeigneten Expressionswirt charakterisiert.

Mit Vorteil kodiert die Nukleinsäure für das Protein MHP 378 aus *Mycoplasma hyopneumoniae* und enthält die Mutationen A629G, A761G und A1757G. Die Nukleinsäure kodiert insbesondere für den Carboxyterminus von MHP 378 beginnend an der Aminosäureposition 286 (Nukleinsäureposition 856, Seq. ID Nr. 1). Die Nukleinsäure kodiert auch vorteilhafterweise für das Protein MHP 651 aus *Mycoplasma hyopneumoniae* und weist die Mutationen A731G, A931G, A1055G, A1424G und A1811G auf, wobei die Nukleinsäure bevorzugt für den Carboxyterminus von MHP 651 beginnend an der Aminosäureposition 351 (Nukleinsäureposition 1051, Seq.ID Nr.2) kodiert.

Die Nukleinsäure wird bevorzugt zur Herstellung rekombinanter Proteine, insbesondere in *E. coli* und / oder *Actinobacillus pleuropneumoniae,* verwendet.

Ein rekombinanter DNA - Vektor umfasst mit Vorteil mindestens eine Nukleinsäure, wobei der Vektor insbesondere in einer Wirtszelle vorliegt. Zur Herstellung eines rekombinanten Proteins wird mit Vorteil die den DNA-Vektor enthaltende Wirtszelle unter Bedingungen zur Expression des jeweiligen immunogenen Protein kultiviert und das Enzym aus der Wirtszelle gewonnen.

Mit Vorteil wird das rekombinante Protein zur Detektion von natürlich infizierten und / oder geimpften Tieren sowie als Impfstoff verwendet.

Die Erfindung wird nachfolgend unter Bezugnahme auf mehrere Ausführungsbeispiele und Figuren näher erläutert. Es zeigen:

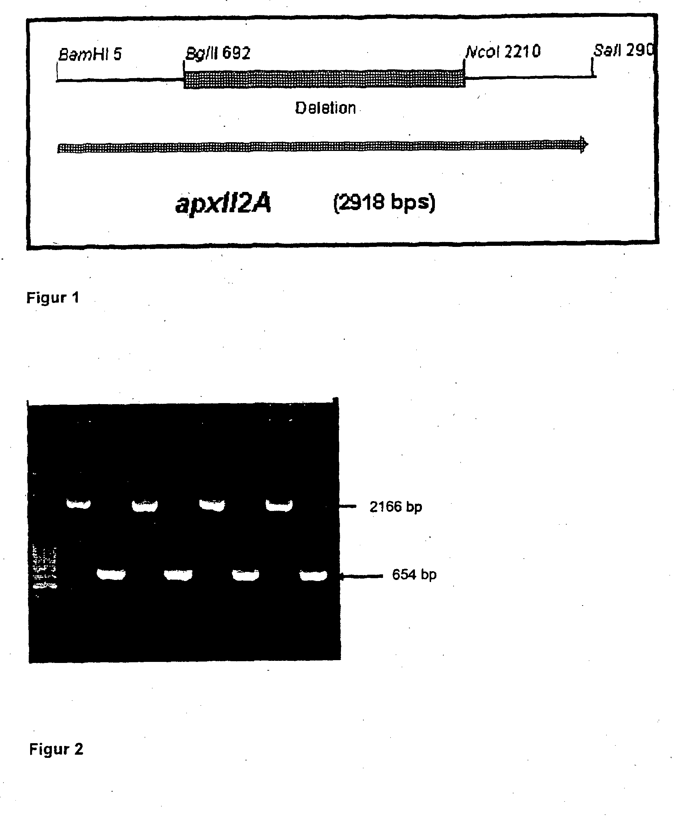

- Figur 1: Deletion in *apxIIA* umfassend 1518 Basenpaare
- Figur 2: PCR - Analyse von erzeugten Mutanten Stämmen Δ*apxIIA* mit internen Primern, mit Spur 1, A. *pleuropneumoniae* Serotyp 1 Wildtyp; Spur 2, Serotyp 1 Δ*apxIIA;* Spur 3, A. *pleuropneumoniae* Serotyp 2 Wildtyp; Spur 4, Serotyp 2 Δ*apxIIA;* Spur 5, A. *pleuropneumoniae* Serotyp 5 Wildtyp; Spur 6, Serotyp 5 Δ*apxIIA;* Spur 7,A. *pleuropneumoniae* Serotyp 9 Wildtyp; Spur 8, Serotyp 9 Δ*apxIIA* und Spur M Marker

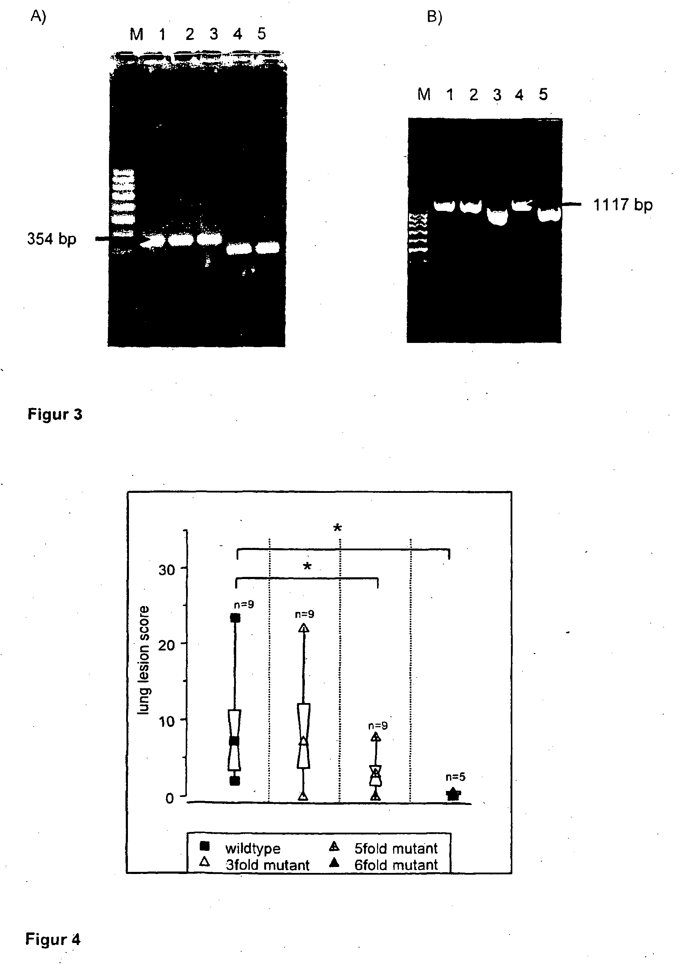

- Figur 3: PCR - Analyse der konstruierten drei-, vier- und fünffach Mutanten, wobei die Deletionen in *aspA* (A) und in *hybB (B)* mit Primern nachgewiesen wurden, die außerhalb des deletierten Genbereichs liegen. Spur 1 zeigt jeweils A. *pleuropneumoniae* Serotyp 2 Wildtyp; Spur 2, Serotyp 2 Dreifachmutante; Spur 3, Serotyp 2 Vierfachmutante *(*Δ*hybB);* Spur 4, Serotyp 2 Vierfachmutante (Δ*aspA*); Spur 5, Serotyp 2 Fünffachmutante und Spur M den Marker.
- Figur 4: "Lung lesion Score" von experimentell infizierten Schweinen, dargestellt als "nodged boxes", dabei repräsentiert die zentrale Einziehung in jeder Box das geometrische Mittel, die obere und untere Begrenzung dieser Box zeigen jeweils die Mitte der Werte in jeder Datenhälfte und die obersten und untersten Punkte entsprechen den minimalen bzw. maximalen Werten

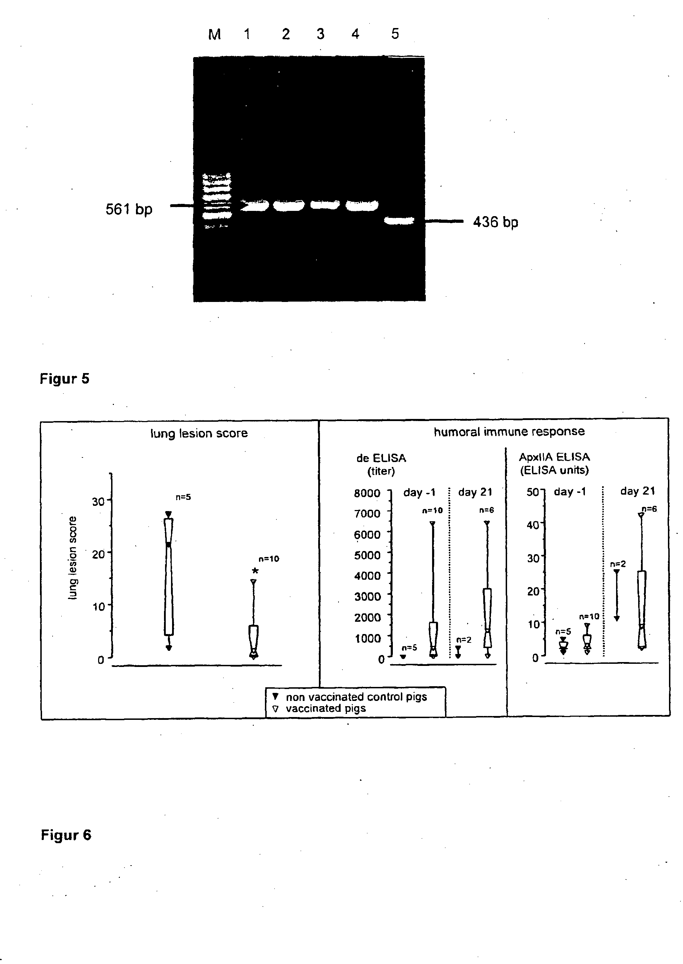

- Figur 5: PCR - Analyse des *fur*-Genes in den konstruierten drei- bis sechsfach Mutanten mit internen Primern, wobei für die sechsfach Mutante mit einer Deletion im *fur* Gen Primer verwendet wurden , die außerhalb des deletierten Bereiches des *fur*-Gens liegen; mit Spur 1, *A. pleuropneumoniae* Serotyp 2 Wildtyp; Spur 2, Serotyp 2 Dreifachmutante; Spur 3, Serotyp 2 Vierfachmutante (Δ*hybB*); Spur 4, Serotyp 2 Fünffachmutante ; Spur 5, Serotyp 2 Sechsfachmutante mit Deletion im *fur*-Gen und Spur M, Marker.
- Figur 6: Pathologische und serologische Ergebnisse nach heterologer Belastung. A) Lunengenläsionenanteil in Form von "nodged boxes" ist gezeigt für alle Schweine. Vier Schweine der mit der Sechsfachmutante geimpften Gruppe wurden am Tag 7 nach der Infektion getötet, alle weiteren überlebenden Schweine am Tag 21 nach der Infektion. Der Stern zeigt einen statistisch signifikanten Unterschied (p < 0.05) im Wilcoxon-Test an. B) Humorale Immunantwort der Kontrollschweine und der geimpften Schweine einen Tag vor und 21 Tage nach der Infektion ermittelt unter Verwendung eines Detergensextrakts (deELISA) oder des rekombinanten ApxIIA-Proteins (ApxII-ELISA) als Festphasenantigene. Die Immunantwort für den ApxIIA-ELISA ist in ELISA Units basierend auf einem externen Standard dargestellt. Aktivitäten mit größer gleich 25 ELISA Units in einem standardisierten ApxII-ELISA wurden als positiv angesehen. Für den deELISA wurde die Immunantwort als Serumtiter im Vergleich zu einer internen Kontrolle angegeben.

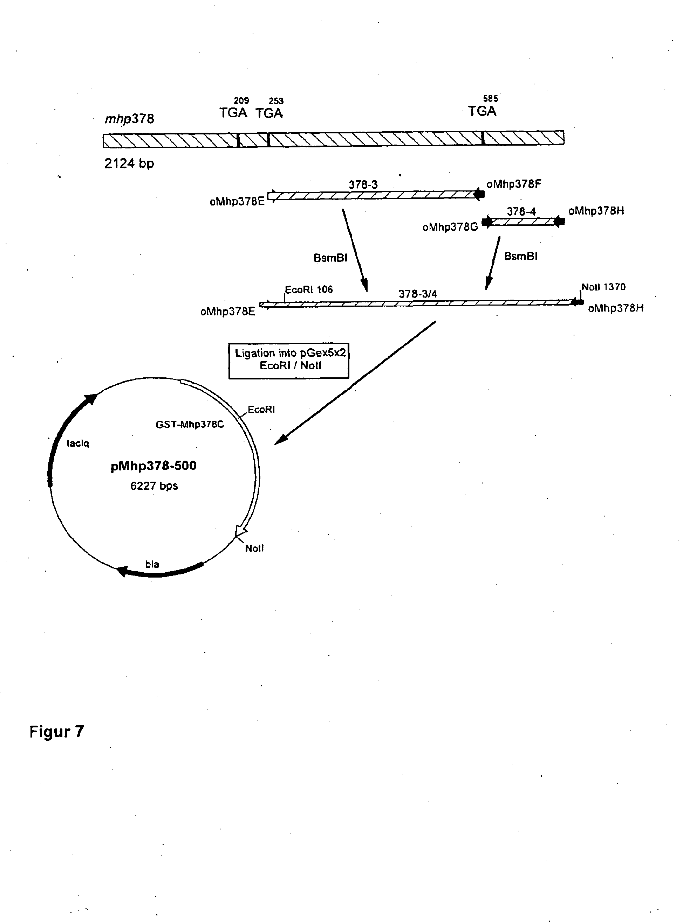

- Figur 7: Schematische Darstellung der Konstruktion des mhp378 Expressionsvektors

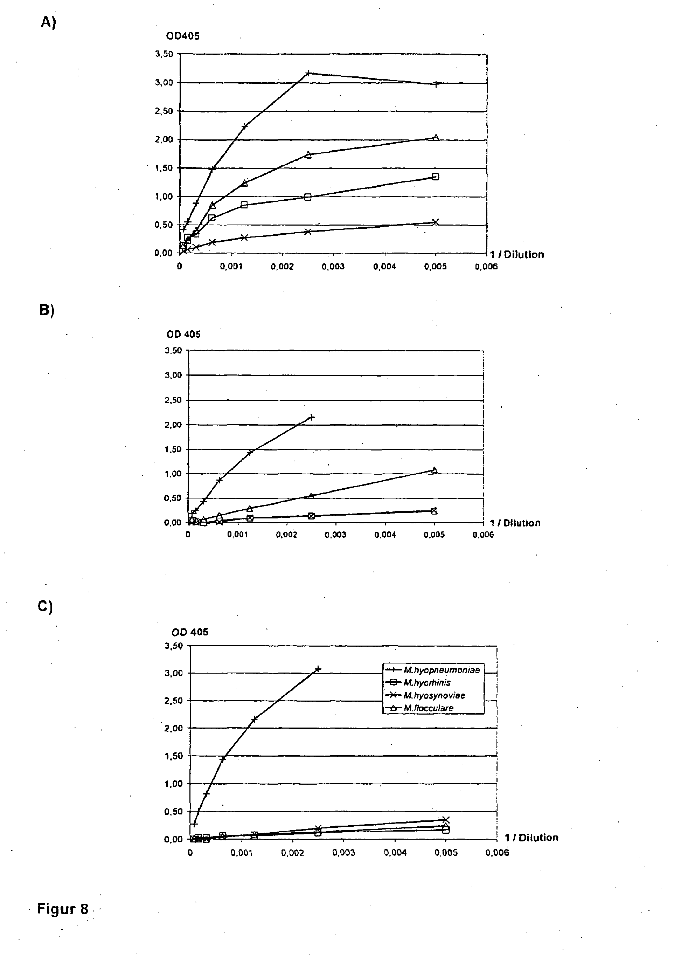

- Figur 8: Reaktivität der GST-Mhp378-C und GST-Mhp651-C Fusionsproteine mit Kaninchen-Hyperimmunseren gerichtet gegen *M. hyopneumoniae, M. hyorhinis, M. hyosynoviae* and *M. flocculare.* A) Kommerzielle ELISA-Platten (IDEXX HerdChek *Mycoplasma hyopneumoniae,* IDEXX Laboratories) beschichtet mit dem vollständigem *M. hyopneumoniae* Zellantigen. B) Polysorb^{®} 96-Mikrotiterplatten (Nunc, Roskilde, Denmark) beschichtet mit GST-Mhp651-C Fusionsprotein und C) mit GST-Mhp378 Fusionsprotein.

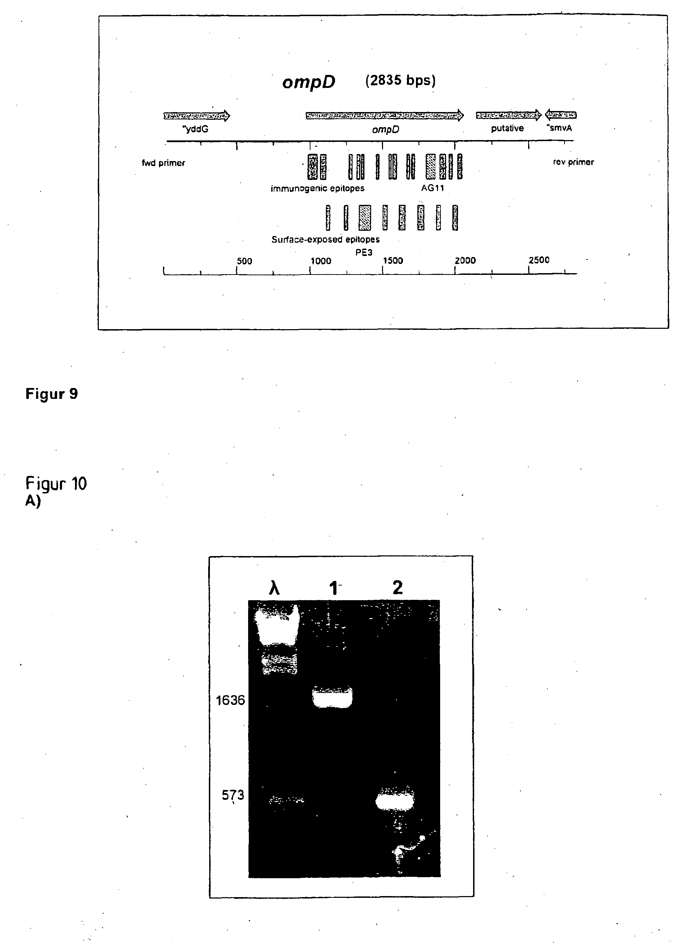

- Figur 9: Schematische Darstellung der berechneten Struktur des OmpD Proteins in Relation zum Genotyp. Antigene Epitope (blau) / Oberflächen-exponierten Bereiche (Grün).

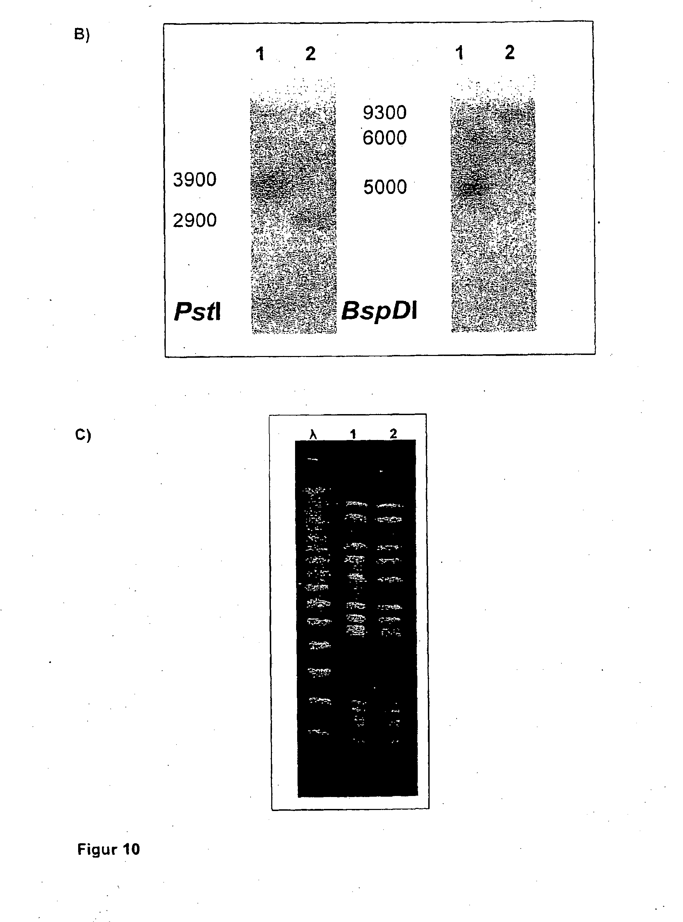

- Figur 10: Überprüfung der ompD Mutation via PCR (A), Southern blot nach Pstl und BspDI Verdau (B) and pulsed field electrophoresis (PFGE) nach Xbal Verdau (C) mit Spur 1 Wild-Typ und Spur 2 *ΔompD* Mutant

### Ausführungsbeispiel 1

### 1.1. Konstruktion von ApxII-Toxin negativen Mutanten von A. pleuropneumoniae Serotyp 1, 2, 5 und 9 zur Verwendung als Tot-Negativmarker-Impfstoff

Das *apxIIA*-Gen kodiert für das ApxIIA Toxin, ein sezerniertes, stark immunogenes Toxin, das bei allen Serotypen von *A. pleuropneumoniae* mit Ausnahme von Serotyp 10 vorkommt. Durch die Deletion sollen Tiere, die mit dem Markerimpfstoff geimpft wurden, serologisch von Tieren, die mit dem Wildtyp infiziert wurden, unterschieden werden können. Ein ELISA, mit dem Antikörper gegen das ApxIIA Toxin nachgewiesen werden, ist bereits im Einsatz.

Die Deletion erfolgte unter Verwendung des etablierten und patentierten "single-step-transconjugation" Systems (Oswald, Tonpitak et al., 1999, A single-step transconjugation system for the introduction of unmarked deletions into Actinobacillus pleuropneumoniae serotype 7 using a sucrose sensitivity marker. FEMS Microbiol.Lett. 179, 153-160). Hierzu wurde das verkürzte *apxIIA-Gen (Dapx2A)* aus dem Serotyp 2 in den Konjugationsvektor pEMOC2 (Baltes et al., 2003, Actinobacillus pleuropneumoniae serotype 7 siderophore receptor FhuA is not required for virulence. Fems Microbiology Letters 220, 41-48) eingefügt. Dieser Vektor enthält ein Chloramphenicol-Resistenzgen und ist nicht in der Lage, in *A. pleuropneumoniae zu* replizieren. Um die Klonierungsarbeiten mit den zur Verfügung stehenden Konjugationsvektoren zu erleichtern, wurden zu Beginn der Arbeiten der Vektor pEMOC2 sowie der patentierte Konjugationsvektor, pBMK1, der ein KanamycinResistenzgen trägt, vollständig sequenziert. Die Sequenzdaten sind unter den Zugangsnummern AJ868289 (pBMK1) und AJ868288 (pEMOC2) in Genbank veröffentlicht worden.

Das pEMOC2 Derivat, dass das verkürzte *apxIIA* Gen (Δ*apxIIA*) trägt, wurde als pAPX700 bezeichnet und mittels Konjugation aus dem E. *coli -* Stamm β2155 *(dapA)* in die entsprechenden *A. pleuropneumoniae* Stämme übertragen. Dort sollte dieses Konstrukt durch homologe Rekombination in die genomische DNA integriert werden ("single crossing over"). Entstehende Chloramphenicol-resistente Kolonien wurden im zweiten Schritt, der Gegenselektion, in einem Medium mit Saccharose kultiviert. Auf dem Konjugationsvektor befindet sich zusätzlich das sacB-Gen. Dieses kodiert für das Enzym Levansucrase, das in gram-negativen Bakterien eine Empfindlichkeit gegenüber Saccharose vermittelt. Durch die Anzucht in dem saccharosehaltigen Medium werden somit diejenigen Bakterien begünstigt, die den Vektor durch eine zweite homologe Rekombination wieder verloren haben. Bei dieser Gegenselektion wird entweder das mutierte Gen oder das Wildtypgen deletiert, sodass im Falle der Deletion des Wildtypgens isogene Mutanten entstehen, die keine Fremd-DNA enthalten.

Für den A. *pleuropneumoniae* Serotyp 2 Stamm konnte die Deletion des *apxIIA*-Gens sofort erfolgreich durchgeführt werden. Bei den Serotypen 5 und 9 konnten zunächst kein "single-crossing-overs" erzeugt werden. Ursächlich hierfür waren wahrscheinlich geringfügige Sequenzunterschiede zwischen den *apxIIA* Genen der Serotypen. Daher wurden für die A. *pleuropneumoniae* Serotypen 5 und 9 serotypspezifische Konjugationsvektoren, die das *apxIIA* Gen des jeweiligen A. *pleuropneumoniae* Serotyps enthalten, konstruiert (Figur 1). Die Plasmide wurden als pAPX705 (Serotyp 5) und pAPX709 (Serotyp 9) bezeichnet.

Mit dem serotypspezifischen Konjugationsvektor für A. *pleuropneumoniae* Serotyp 5 konnte eine isogene ApxIIA-Mutante für diesen Serotyp erzeugt werden. Bei *A. pleuropneumoniae* Serotyp 9 konnten zwar Chloramphenicol resistente Kolonien generiert werden. Diese revertierten bei der Gegenselektion aber immer wieder zum Wildtyp.

Da zur Lösung der Probleme bei der Mutagensierung von A. *pleuropneumoniae* Serotyp 9 umfangreichere Arbeiten nötig waren, wurde zunächst ein A. *pleuropneumoniae* Serotyp 1 Stamm als alternative Komponente für den Tot-Negativmarker-Impfstoff ausgewählt. Dieser Serotyp ist in Nordamerika der am häufigsten vorkommende Serotyp, während der Serotyp 9 überwiegend für Deutschland beschrieben ist und auch hier nur für einen relativ kleinen Teil der Erkrankungen verantwortlich ist.

Da laut Genombankrecherche die *apxIIA*-Gene von A. *pleuropneumniae* Serotyp 1 und 5 in den Bereichen, in denen im Verlauf des Verfahrens eine homologe Rekombination stattfindet, identisch sind, wurde der spezifische Konjugationsvektor für A. *pleuropneumoniae* Serotyp 5 für die Erstellung einer isogenen Deletionmutante von A. *pleuropneumoniae* Serotyp 1 verwendet. Auf diese Weise konnte für den *A. pleuropneumoniae* Serotyp 1 erfolgreich ein "double-crossing-over", d.h. eine isogene Mutante erzeugt werden. Die so konstruierten Mutanten der *A. pleuropneumoniae* Serotypen 1, 2 und 5 wurden dann zur Herstellung eines Subunit-Impfstoffes eingesetzt. Parallel hierzu wurde im Laufe des letzten Jahres ebenfalls die Mutagenisierung des A. *pleuropneumoniae* Serotyp 9 erfolgreich abgeschlossen. Die *apxIIA* Mutanten aller vier Serotypen wurden durch PCR Analyse (Figur 2), Southern Blot Analyse nach Restriktionsenzymverdau von chromosomaler DNA, Pulsfeldgelelektrophorese sowie DNA-Sequenzierung als isogene, Fremd-DNA freie Mutanten charakterisiert. Außerdem wurde die Ausschaltung des ApxIIA Toxins durch Western Blot Analyse nach SDS-Polyacrylamid Gelelektrophorese von Kulturüberständen aller Serotypen bewiesen.

### 1.2. Prüfung der Wirksamkeit eines auf der Basis der Mutanten hergestellten Subunit-Impfstoffes im Schwein und Entwicklung eines A. pleuropneumoniae-Tot-Negativmarker-Impfstoffes

Aus den erzeugten Mutanten der *A. pleuropneumoniae* Serotypen 1, 2 und 5 wurde nach der von Goethe et al. (2000; A novel strategy for protective Actinobacillus pleuropneumoniae subunit vaccines: detergent extraction of cultures induced by iron restriction. Vaccine 19: 966-975) beschriebenen und patentierten Methode (Patent DE 197 53 176) ein Inaktivatimpfstoff formuliert. Dieser wurde mittels Western Blot Analyse auf zwei seiner antigenen Proteine hin überprüft und anschließend im Tierversuch getestet. Dabei wurden 24 Schweine im Alter von 4 Wochen genutzt. Die Immunisierungen erfolgen im Alter von 4 und 7 Wochen, wobei 14 Schweine mit dem erzeugten Inaktivat-Markerimpfstoff immunisiert wurden und 10 Schweine als Kontrolltiere dienten und mit einem Placebo, dem die antigenen Komponenten fehlten, geimpft wurden. Anschließend wurden alle Tiere (mit Ausnahme von vier mit dem Inaktivat-Markerimpfstoff geimpften Schweinen) im Alter von 10 Wochen mit einem heterologen *A. pleuropneumoniae* Serotyp 2 Stamm (i.e. einem anderen Serotyp 2 Stamm als dem, der zur Impfstoffherstellung diente) in der Aerosolkammer infiziert. Die vier nicht infizierten Tiere dienten als Kontrollgruppe um sicherzustellen, dass auch im weiteren Verlauf der Haltung keine Antikörper gegen das Apxll-Protein gebildet werden. Die eine Hälfte der Tiergruppen wurde nach 7 Tagen und der Rest nach 21 Tagen euthanasiert und pathologisch sowie bakteriologisch untersucht.

Im Verlaufe dieses Versuches konnte ein sehr guter Schutz sowohl gegenüber klinischen als auch gegenüber pathologischen Symptomen für Tiere nachgewiesen werden, die mit dem Inaktivat-Markerimpfstoff immunisiert wurden. Acht von zehn geimpften Tieren hatten keinerlei pathologischen Veränderungen, während alle Kontrolltiere z.T. sehr starke Läsionen in den Lungen aufwiesen. Außerdem konnten mit Hilfe des ELISAs Antikörper gegen das ApxIIA-Toxin detektiert werden. Der zur Belastung genutzte *A. pleuropneumoniae* Serotyp 2 konnte in geimpften Schweinen signifikant weniger häufig nachgewiesen werden als in ungeimpften Schweinen der Kontrollgruppe (Wilcoxontest, p < 0,05).

Da ein Impfstoff für *A. pleuropneumoniae* zusätzlich zu den im Impfstoffe enthaltenen Serotypen auch noch gegen weitere Serotypen schützt sollte, wurde ein weiterer Tierversuch durchgeführt. Dabei wurden 15 Schweine in 2 Gruppen aufgeteilt; eine Gruppe von 10 Tieren wurde mit dem Inaktivat-Markerimpfstoff, bestehend aus den Serotypen 1, 2 und 5 immunisiert, die andere Gruppe mit 5 Tieren mit einem Placebo. Die Immunisierungen erfolgten im Alter von 4 und 7 Wochen. In der 10. Lebenswoche wurden die Tiere dann mit einem *A. pleuropneumoniae* Serotyp 9 Stamm infiziert. Die Euthanasie und die Sektionen erfolgten 7 Tage bzw. 21 Tage der Belastung. Die Ergebnisse nach der Belastung zeigen einen sehr guten serotypübergreifenden Schutz des Impfstoffes hin. So entwickelten alle Tiere aus der Kontrollgruppe Dyspnoe, Anorexie und Depressionen und verstarben innerhalb von 48 Stunden nach der Belastung. Die immunisierten Tiere zeigten keinerlei klinische Symptome und die Körpertemperatur überstieg nicht 40.1°C. Die pathologische Untersuchung der nicht vakzinierten Kontrollgruppe zeigte eine starke fibrinogene Pleuritis verbunden mit einer Pneumonia, die einen großen Teil der Lunge betraf. Geimpfte Tiere hingegen zeigten keine Veränderungen des Rippenfells (Pleura) oder der Lunge. Der verabreichte Belastungsstamm konnte von der nicht-geimpften Kontrollgruppe in großer Zahl reisoliert werden, während dies nur bei zwei der vakzinierten Schweine in geringer Zahl möglich war.

Proben von Seren wurden drei Wochen nach der Immunisierung und drei Wochen nach der Infektion gesammelt und serologisch in zwei unterschiedlichen ELISA-Tests untersucht. Im ApXII_ELISA wird das Apxll Toxin als Festphasenantigen benutzt. Die Reaktion wird in Form von ELISA-Einheiten (EU) basierend auf einem externen Standard quantifiziert, wobei Aktivitäten von kleiner/gleich 10 EU im Serum als negativ, 11 bis 25 EU als fraglich, und Aktivitäten größer 25 EU als positiv betrachtet wurden. Der zweite ELISA-Test, der sog. deELISA, verwendet einen Detergensextrakt von Proteinen, die assoziiert mit der äußeren Membran von *A.pleuropneumoniae* vorliegen, als Festphasenantigen. Im deELISA ist die Immunantwort quantifiziert als der Serumtiter im Vergleich zu einer internen negativen Kontrolle. Die Negativkontrolle besteht aus einer Mischung gleicher Volumina aller Serunproben, die bei Ankunft der nicht-infizierten Schweine genommen wurden. Die Positivkontrolle besteht aus einer Mischung von gleichen Volumina allen Serenproben, die von infizierten Schweinen am Tag 21 nach der Infektion genommen wurden. Der Titer des deELISA ist dabei die höchste Serumverdünnung, die in einer optischen Dichte resultiert, die doppelt so hoch ist wie die des Negativkontrollserums bei einer Verdünnung von 1 zu 100.

Schweine aus der Kontrollgruppe wiesen weder im de-ELISA noch im APXIIa Elisa einen Titer zum Zeitpunkt der Infektion auf. Die vakzinierten Tiere hingegen wiesen zum Zeitpunkt der Infektion und drei Wochen nach der Infektion einen hohen de-ELISA Titer auf, waren aber zu beiden Zeitpunkten im ApxIIA-ELISA negativ.

### Ausführungsbeispiel 2

### 2.1. Entwicklung eines A. pleuropneumoniae Lebend-Negativmarker-Impfstoffes

Ziel ist die Attenuierung eines *A. pleuropneumoniae* Serotyp 2 Stammes durch Deletionen in verschiedenen Virulenz-assoziierten Faktoren. Dadurch soll erreicht werden, dass der Stamm keine klinischen Symptome mehr auslöst, aber nach Aerosolapplikation immer noch eine Immunantwort induziert. Dieser Stamm wird dann als Lebendimpfstoff eingesetzt.

Grundlage für diese Arbeiten bildete ein vorhandener Prototyp eines A. *pleuropneumoniae* Serotyp 2 Lebend-Negativmarker-Impfstoffes (Tonpitak et al., 2002, Construction of an Actinobacillus pleuropneumoniae serotype 2 prototype live negative-marker vaccine. Infect Immun 70, 7120-7125). Dieser enthält zwei attenuierende Deletionen: eine im *apxIIA-Gen,* die hier auch als Negativmarkierung dient und eine im *ureC-Gen,* das für eine Komponente des komplexen Enzyms Urease kodiert. Basierend auf dieser Doppelmutante wurde eine Dreifachmutante mit Hilfe des "single-step-transconjugation" Sysems konstruiert, die eine zusätzliche Deletion im dmsA-Gen enthält. Die erzeugte Dreifachmutante wurde genotypisch und phänotypisch als isogene, Fremd DNA freie Mutante bestätigt und im Tierversuch mit aerogener Belastung auf ihre Virulenz geprüft. Dazu wurden Schweine mit einer Dosis von 1.2 x 10⁵ KBE (koloniebildenden Einheiten) der Dreifachmutante in der Aerosolkammer infiziert. Alle Schweine entwickelten die typischen Anzeichen einer Infektion. Ein Schwein verstarb innerhalb von 2 Tagen und ein weiteres Schwein innerhalb von 9 Tagen nach der Infektion. Die überlebenden Schweine wurden nach 21 Tagen euthanasiert und pathologisch untersucht. Dabei zeigten 4 von 5 Schweinen Lungenläsionen (Figur 4), was für die Verwendung als Impftstoff völlig unakzeptabel ist.

Zur Erzeugung eines Impfstammes mussten also weitere Deletionen eingefügt werden. Als Zielgene hierfür wurden das *aspA Gen* und das *hybB* Gen ausgewählt. Ausgehend von der Dreifachmutante wurden zwei Vierfachmutanten erzeugt, jeweils eine mit zusätzlich deletiertem *aspA* bzw. *hybB* Gen. Außerdem wurde eine Fünffachmutante erstellt, bei der sowohl das *aspA* als auch das *hybB* Gen in inaktivierter Form vorliegen. Diese Mutanten wurden phänotypisch und genotypisch charakterisiert. Dabei könnten die erzeugten Mutanten durch PCR Analyse (Figur 3), Southern-Blot Analysen nach Restriktionsenzymverdau und Pulsfeldgelelektrophorese als isogene, Fremd-DNA freie Mutanten identifiziert werden.

Anschließend wurde die Fünffachmutante in einem Tierversuch auf ihre Virulenzeigenschaften hin überprüft. Nach dem oben erläuterten Schema wurden 9 Tiere in der Aerosolkammer mit dem Erreger in einer Dosis von 9.1 x 10⁴ KBE pro fünf Schweine sowie mit der Fünffachmutante in einer Dosis von 1.5 x 10⁵ KBE pro fünf Schweine belastet. In der Gruppe mit der Fünffachmutante wiesen 6 von 9 Tieren einen Tag nach der Infektion eine erhöhte Körpertemperatur von mehr als 40.5°C auf und 3 von 9 Tieren verweigerten die Nahrungsaufnahme. Drei Tage nach der Infektion lag die Körpertemperatur aller mit der Fünffachmutante geimpften Tiere unter 40.5°C und am Tag 7 nach der Infektion entwickelte nur ein geimpftes Tier Husten. Die Schweine in der Kontrollgruppe hingegen entwickelten schwere Krankheitssymptome wie Anorexie, Lethargie, Atemnot (Dyspnoe) oder Erbrechen am ersten Tag nach der Infektion. Die Tiere wurden nach 7 bzw. 21 Tagen euthanasiert und seziert. Die Tiere, die mit der Fünffachmutante infiziert wurden, hatten dabei signifikant weniger Lungenläsionen (Figur 4), als eine mit dem Wildtypstamm infizierte Kontrollgruppe (Wilcoxontest, p < 0,05).. Alle Tiere zeigten 21 Tage nach der Infektion deutliche Antikörpertiter. Die verursachten Lungenläsionen sind aber immer noch so stark, dass diese Fünffachmutante noch nicht als Impfstamm verwendet werden kann.

Weiterführend Ziel war es daher, die erzeugte Fünffachmutante weiter zu attenuieren. Dazu wurde das *fur* Gen (ferric uptake regulation, *fur)* deletiert. Zur Mutagenisierung musste ein serotypspezifischer Konjugationsvektor konstruiert werden, da die Sequenzunterschiede zwischen den Serotypen 2 und 7 so groß waren, dass das von Jacobsen genutzte Konjugationsplasmid zu keinen "Single-Crossing-Over" beim Serotyp 2 führte. Mit Hilfe des "single-step-transconjugation" Systems konnte dann eine Sechsfachmutante erzeugt werden, die sich in der genotypischen und phänotypischen Charakterisierung als isogene, Fremd-DNA freie Mutante bestätigt hat (Figur 5).

Zur Untersuchung der Virulenzeigenschaften der Sechsfachmutante wurde eine Gruppe von 5 Schweinen mit der Sechsfachmutante in einer Dosis von 1.1 x 10⁵ cfu pro fünf Schweine in einer Aerosolkammer belastet. Vier der fünf mit der Sechsfachmutante belasteten Schweine zeigten einen Tag nach der Infektion eine erhöhte Körpertemperatur von über 40.5°C auf. Weitere Symptome waren nicht detektierbar. Die infizierten Tiere wurden drei Wochen *post infectionem* euthanasiert und pathologisch untersucht. Alle Schweine hatten bei der Sektion nur minimale pathologische Veränderungen (durchschnittlicher Lungenläsionenanteil von 0,3).

Am Tag 21 nach der Infektion konnten die Fünffach- und Sechsfachmutanten lediglich in einer Probe von intaktem Lungengewebe nachgewiesen werden, während der WildtypStamm aus 4 von 5 Proben reisoliert werden konnte.

Schweine infiziert mit jeweils der Fünffach- oder Sechsfachmutante zeigten zu keiner Zeit nach der Infektion einen Antikörpertiter in einem ApxII-ELISA, zeigten aber eine messbare Reaktion in einem deELISA 21 Tage nach der Infektion.

Ein Lebendimpfstoff für *A. pleuropneumoniae,* bestehend aus nur einem Serotyp, hat nur dann eine kommerzielle Zukunft, wenn er zusätzlich zu einer Protektion gegen den eigenen Serotyp auch einen serotyp-übergreifenden Schutz vermitteln kann. Daher wurde auf die Überprüfung eines homologen Schutzes bei Nutzung der Sechsfachmutante als Lebendvakzine verzichtet. Stattdessen wurde ein Tierversuch mit heterologer Belastung nach Immunisierung durchgeführt.

Dabei sind 10 Schweine im Alter von 7 Wochen mit der Sechsfachmutante per Aerosolapplikation immunisiert worden, während 5 Kontrolltiere nur mit Kochsalzlösung (150 mM) behandelt wurden. Beide Gruppen wurden anschließend mit einem A. *pleuropneumoniae* Serotyp 9 infiziert. Die Gruppen wurden wieder 7 Tage bzw. 21 Tage nach der Belastung euthanasiert und seziert. Ergebnisse zeigen eine deutliche serotypübergreifende Wirkung des Lebendimpfstoffes. So verstarben 3 der 5 Tiere aus der Kontrollgruppe während alle Tiere der geimpften Gruppe keine oder geringe klinische Symptome entwickelten. Die vakzinierten Schweine wiesen eine deutlich geringere Lungenläsionsrate auf als die Kontrolltiere (Figur 6). Der Impfstamm konnte nur sporadisch isoliert werden, während bei der Reisolierung des Wildstammes keine Unterschiede zwischen der geimpften und der Kontrollgruppe auftraten (Tabelle 1). Drei Wochen nach der Immunisierung wurden alle Schweine im deELISA und ApxII-Elisa getestet. Die Kontrolltiere zeigten in beiden Tests einen negativen Titer, während die geimpften Tiere einen nachweisbaren Antikörpertiter im deELISA-Test jedoch nicht im ApxII-ELISA aufwiesen (Tabelle 1). Drei Wochen nach der Infektion zeigten die zwei überlebenden Tiere der Kontrollgruppe einen Antikörper-Titer im deELISA und ein Tier war positiv im ApxII-ELISA.

Die geimpften Tiere zeigten starke Titer im deELISA und zwei waren positiv im ApxII-ELISA.

**Tabelle 1**

| **Gruppe** | **Infektionsdosis (cfu per 5 pigs)^{a}** | **Zeit der Nekroskopie** | **Serologische Reaktion** | | **Anzahl der Tiere mit Lungenläsionen^{d}** | **Arithmietisc hes Mittel ± Standardabweichung der Lunenläsionsrate** | **Anzahl der Tiere und Lokalisierung der reisolierten *A. pleuropneumoniae* in der post mortem Analyse** | | | | **Arithmietisch es Mittel ± Standardabweichung der Reisolationsrate** |
|---|---|---|---|---|---|---|---|---|---|---|---|
| | | | Detergentien - Spülung^{b} | ApxIIA^{c} | | | Mandel | Lymphknoten | Lunge | | |
| | | | | | | | | | pneumonisch | Intakt | |
| geimpft^{e} | 1,6 x 10⁵ | Tag -1 | 1140±1946 | 3,8±2,9 | | | | | | | |
| | | Tag 7 | 950±755 | 4,5 ±3,7 | 3/4 | 4,9±5,4 | 1/4 | 4/4 | 3/3 | 3/4 | 1,5±1 |
| | | Tag 21 | 2067±2406 | 14,7±16, 1' | 4/6 | 3±5,6 | 2/6 | 1/6 | 2/4 | 2/6 | 1,4±2,8 |
| Nicht geimpfte Kontrollgruppe | 1.7 x 10⁵ | Tag -1 | 0 | 2,8±1,6 | | | | | | | |
| | | Tag 2 | | | 3/3 | 24,9±3,1 | 3/3 | 3/3 | 3/3 | 3/3 | 7 |
| | | Tag 21 | 200±283 | 18±9,9 | 2/2 | 3±1,6 | 1/2 | 0/2 | 1/2 | 1/2 | 0,5±0,7 |

| | | | | | | | | | | | |
|---|---|---|---|---|---|---|---|---|---|---|---|
| ^{a} *A. pleuropneumoniae* Serotyp 9 wurde bis zu einer optischen Dichte von 0.43 bei 600 nm angezogen und dann 1 : 30,000 mit steriler 0.9% NaCl-Lösung verdünnt. 13 ml dieser Lösung wurden in die Aerosolkammer gesprüht. Die angegebene Zahl sind die Kolonienbildenden Einheiten in 13 ml. ^{b} Die Festphasenantigene wurden wie bereits beschrieben (Goethe et al., 2000) vorbereitet. Die angegebene Nummer stellt das arithmethische Mittel der höchsten Serumverdünnung dar, die in einer optischen Dichte resultiert, die doppelt so hoch ist wie die Negativkontrolle des Serums bei einer Verdünnung von 1 : 100. ^{c} *Rekombinantes* ApxIIA Protein wurde als Festphasenantigen verwendet. Die Zahl stellt das arithmetische Mittel der Serumaktivität in ELISA Units dar. ^{d} Die Lungenläsionsrate wurde nach Hannan et al. (Res. Vet.Sci., 1982, 33:76-88) ermittelt. ^{e} Die *A. pleuropneumoniae -* Sechsfachmutante wurde bis zu einer optischen Dichte von 0.34 bei 600 nm angezogen und 1 : 30,000 mit einer sterilen 0.9% NaCl-Lösung verdünnt. 13 ml dieser Lösung wurden in die Aerosolkammer gesprüht. | | | | | | | | | | | |

### Ausführungsbeispiel 3

### 3.1. Identifizierung, Klonierung und Expression immunogener Proteine aus M. hyopneumoniae

Da die Membranfraktion die meisten immunogenen Proteine enthält, wurden zur Identifizierung neuer immunogener Proteine von *M. hyopneumoniae* hauptsächlich Membranfraktionen von Zellen, die unter Labor-Standardbedingungen gewachsen waren, hergestellt. Die Membran-Proteine und Membran-assozierten Proteine wurden mittels Triton X114 Extraktion gewonnen (Regula et al., Towards a two-dimensional proteome map of Mycoplasma pneumoniae, Electrophoresis, 2000, 21, 3765-3780).

Anschließend wurden die Membran-Extrakte in der 2-D Gelelektrophorese aufgetrennt und mit anschließendem Western-Blot untersucht, wobei verschiedene Fokussierungsbedingungen wie pH-Gradient und Gel-Konzentrationen getestet wurden. Zur Detektion möglicher Kandidatenproteine wurde ein Rekonvaleszentrenserum verwendet. Hierbei handelt es sich um einen Pool von fünf Einzelseren von ungeimpften Schweinen, bei denen eine Infektion mit *M. hyopneumoniae* vorlag. Zum Vergleich wurden die Proben auch mit einem Hyperimmunserum, das unter Verwendung des Impfstoffes "Respiporc M.hyo 1 Shot" vom Impfstoffwerk Dessau-Thornau (IDT, Deutschland) hergestellt wurde, geblottet. Hierdurch wurden solche Spots gefunden, die nur oder zumindest deutlich stärker mit dem Rekonvaleszentenserum reagieren.

Aus einem Coomassie Blau-gefärbten Gel wurden mehrere Protein-Spots ausgestochen, von denen die Spots Nr. 43 und Nr. 48 identifiziert wurden. Zur Identifizierung wurden die Spots mit Trypsin verdaut und die hierbei entstandenen Peptide wurden mit Hilfe eines Q-Tof MS analysiert.

Spot 43 wurde als hypothetisches Lipoprotein Mhp378 identifiziert. Das Protein zeigt die höchsten Homologien zu hypothetischen Lipoproteinen anderer Mycoplasmen-Spezies. Hinweise auf mögliche Funktionen dieser Proteine gibt es bisher nicht. Für weitere Arbeiten, wie der Etablierung eines ELISA-Tests zur Detektion von natürlich infizierten Tieren, werden größere Mengen an rekombinantem Protein benötigt. Hierzu wurden Expressionsvektoren zur Herstellung von GST-Fusionsproteinen konstruiert.

Das dem Lipoprotein Mhp378 entsprechende Gen *mhp378* kodiert für ein 708 Aminosäuren großes Protein und enthält drei TGA-Kodons an den Aminosäurepositionen 209, 253 und 585. Auf Grund der spezifischen Kodonnutzung in Mycoplasmen fungieren diese Tripletts nicht als Stop-Kodon, sondern kodieren für Tryptophan. Zur heterologen Expression des mhp378 Gens in *E. coli* wurde durch PCR - Mutagenese nach der Methode von Shigaki und Hirschi (2001,Use of class IS restriction enzymes for site-directed mutagenesis: Variations on Phoenix mutagenesis; Analytical Biochemistry 298:118-120) das TGA ⁵⁸⁵ Kodon zu TGG mutiert. Hierzu wurde der Bereich zwischen den Aminosäurepositionen 253 und 585 mit Hilfe der PCR-Primer oMHP378E und oMHP378F amplifiziert und der Bereich zwischen den Aminosäurepositionen 585 und dem Ende des Leserahmens mit den Primern oMHP378G und oMHP378H amplifiziert. Im Überlappungsbereich wurden beide Fragmente mit dem Restriktionsenzym BsmBI geschnitten und ligiert. Das resultierende Fragment kodiert für den Carboxyterminus des Proteins (Aminosäure 286 bis 708) und wurde in den Expressionsvektor pGEX5-3 kloniert (Figur 7). Der so konstruierte Expressionsvektor pMhp378-500 ermöglicht in *E. coli DH5α* die induzierbare Synthese eines GST-Mhp378^{(AS286-708)} Fusionsproteins. Das exprimierte Protein wurde in *E. coli* DH5α als unlösliches Aggregat vor. Durch Behandlung mit einem 5 M Harnstoffpuffer wurde das aggregierte Fusionsprotein weitgehend gereinigt.

Das das Lipoprotein Mhp651 kodierende Gen *mh*p651 enthät 5 TGA-Kodons an den . Aminosäurepositionen 243, 309, 351. 474 und 603. Zur weiteren Untersuchung wurden zwei Expressionsvektoren analog dem für mhp378 beschriebenen Verfahren konstruiert.

Zur Expression des Aminoterminus mit den Aminosäuren 19 bis 210 wurde der Bereich des mhp651 Gens zwischen den beiden EcoRI Schnittstellen an Nukleotidpositionen 53 und 623 amplifiziert, geschnitten und in den Expressionsvektor pGEX5-2 kloniert. Der so konstruierte Expressionsvektor pMhp651-N^{(AS 19 bis 210)} ermöglicht in *E. coli* DH5α die induzierbare Synthese eines GST-Fusionsproteins mit dem aminoterminalen Bereich des Mhp651-Proteins.

Zur Expression des Carboxyterminus (Aminosäuren 351 bis 714) wurden die TGA Kodons an den Positionen 47 und 603 nach dem oben beschriebenen Verfahren zu TGG mutiert. Hierfür mussten die PCR-Fragmente amplifiziert und mit BsmBI geschnitten, ligiert und in den Expressionsvektor kloniert werden. Der konstruierte Expressionsvektor pMhp651-C ^{(AS351-714)} enthält den carboxyterminalen Bereich des Proteins.

Zur Gewinnung der GST-Fusionsproteine wurden die entsprechenden *E. coli* DH5α Stämme in Volumina bis zu 200 ml kultiviert. Die Induktion der Expression und die Reinigung der Fusionsproteine per Affinitätschromatographie erfolgten gemäß der Angabe des Herstellers (Amersham Biosciences). Die zwei Fusionsproteine GST-Mhp651-N^{(AS19-210)} und GST-Mhp651-501-C^{(AS351-714)} wurden als lösliche Proteine in *E. coli* DH5α exprimiert und gereinigt.

Die gereinigten Fusionsproteine GST-Mhp378^{(AS286-708)}, GST-Mhp651-N^{(AS19-210)} und GST-Mhp651-C^{(AS351-714)} wurden anschließend in Western-Blot Experimenten auf ihre Reaktion mit dem Rekonvaleszentenserum und dem Hyperimmunserum geprüft. Alle drei Proteine zeigen eine positive Reaktion mit beiden Seren, wobei die Intensität der Wechselwirkung mit dem Rekonvaleszentenserum deutlich stärker ist. Das als Kontrolle benutzte GST-Protein reagierte mit keinen der beiden Seren. Die beiden auf Grund der Genomsequenz vorhergesagten Gene *mhp378* und *mhp651* von *M. hyopneumoniae* werden somit *in vivo* exprimiert und die entsprechenden Proteine sind immunogen.

Zur weiteren Charakterisierung wurden die rekombinanten Fusionsproteine GST-Mhp378 ^{(AS286-708)} und GST-Mhp651-C^{(AS351-714)} als Festphasenantigen auf Polysorb^{®} ELISA-Platten aufgebracht. Zuerst wurden mögliche Kreuzreaktionen mit den anderen beim Schwein vorkommenden *Mycoplasma* Spezies getestet. Hierzu wurden ELISA-Platten mit polyklonalen Kaninchen-Seren inkubiert, die gegen Gesamtzellpräparationen von M. *hyorhinis, M. hyosynoviae* und *M. flocculare* gerichtet sind.

Die Fusionsproteine wurden dafür zunächst in einem Carbonat-Puffer (50 mM, pH 9.6) gelöst und die optimale Beschichtungskonzentration mittels Checkerboard-Titration unter Verwendung des Schweine- Rekonvaleszentenserums ermittelt, wobei sich eine optimale Konzentration von 2.5 µg/ml ergab.

Anschließend wurden Polysorb^{®} 96-Mikrotiterplatten (Nunc, Roskilde, Dänemark) mit 100 µl der verdünnten Proteinlösung pro Vertiefung bei 4°C übernacht ohne anschließendes Blocken beschichtet. Als Kontrolle wurde eine ELISA-Platte (IDEXX HerdCheck M. hyopneumoniae) verwendet, die mit dem kompletten *M. hyopneumoniae* Zell-Antigen beschichtet ist. Die verdünnten Seren und die Ziegen-anti-Schwein- oder Ziegen-anti-Kaninchen-Peroxidase-Konjugate (Dianova, Hamburg, DE) wurden jeweils für eine Stunde bei Raumtemperatur inkubiert. Der ELISA wurde anschließend unter Verwendung von ABTS (2,2'-Azino-di-[3-ethylbenzithiazolinsulfonat]) entwickelt und die Zunahme der Farbintensität bei einer OD von 405 nm gemessen.

Die experimentellen Ergebnisse zeigen eine starke Kreuzreaktion der Kaninchen-Hyperimmunseren gerichtet gegen *M.flocculare* und *M.hyorhinis* auf der kommerziell erhältlichen ELISA-Platte (Figur 8A). Von den zwei getesteten Fusionsproteinen zeigte lediglich das GST-Mhp651-C^{(AS352-714)} -Fusionsprotein eine Kreuzreaktion mit dem für *M.flocculare* spezifischen Hyperimmunserum (Figur 8B), während das GST- Mhp378^{(AS254-708)},- Fusionsprotein lediglich von dem für *M.hyopneumoniae* spezifischen Immunserum erkannt wurde (Figur 8C). Diese Experimente zeigen, dass die aus M. *hyopneumoniae* isolierten immunogenen Proteine Mhp378 und Mhp651 als spezifische Marker zur Detektion von mit *M. hopyneumonmiae* infizierten Nutztieren bzw, geimpften und natürlich infizierten Nutztieren verwendet werden können ohne dass es zu falsch positiven Reaktionen - bedingt durch eine Besiedlung/Infektion der Schweine mit anderen Mykoplasmen - kommt.

### Ausführungsbeispiel 4

### 4.1. Identifizierung, Klonierung und Expression immunogener S. Typhimurium Proteine

Für die Identifizierung immunogener S. Typhimurium Proteine wurden oberflächenassoziierte Proteine über eine im Labor des Anmelders etablierte "Detergent Wash"-Methode isoliert und mittels 2D-Gelelektrophorese in Kombination mit Western-Blotting detektiert. Hierbei wurden Proteine zunächst nach ihrem isoelektrischen Punkt und im Anschluss nach ihrer elektrophoretischen Beweglichkeit auf einem SDS-PAGE Gel aufgetrennt und anschließend mittels einer Semi-Dry Apparatur auf eine Nitrozellulose-Membran transferiert. Mittels dreier Seren wurden die transferierten Proteine auf ihre Immunogenität hin überprüft.

Serum 1 ist ein porcines Impfserum, das nach mehrfacher Immunisierung von Schweinen mit dem attenuierten Lebendimpfstoff SALMOPORC^{®} (Hersteller: Impfstoffwerk Dessau, Deutschland) gewonnen wurde. Serum 2 ist ein Seren-Pool von Tiere, die nicht gegen S. Typhimurium geimpft wurden sondern bei der Fleischsaftserologie als Salmonellenantikörper-positiv auffielen. Serum 3 ist ein Seren-Pool von insgesamt 120 Tieren, die im Rahmen eines Fütterungsversuches mit S. Derby infiziert worden waren. Neben der Betrachtung differentiell exprimierter Proteine nach Anzucht unter standardisierten Laborbedingungen und unter Eisenmangelbedingungen wurde primär nach solchen Proteinen gesucht, die sowohl vom Impfserum als auch von den beiden Rekonvaleszentenseren als immunogen erkannt wurden.

Auf diese Weise wurde das Protein DWST-5 (outer membrane porin NmpC / OmpD precursor) als differentiell exprimiert identifiziert. Das Protein DWST-5 wurde mittels Datenbankrecherche auf seine Eignung für die Entwicklung eines Lebend-Negativmarker-Impfstoffes überprüft. Ein geeignetes Protein muss eine hohe Spezifität für Salmonellen aufweisen, so dass Kreuzreaktionen mit Antikörpern gegen andere Enterobakterien möglichst vermieden werden, wenn es als Festphasenantigen in einem ELISA System eingesetzt wird. Das als outer membrane porin D (OmpD) identifizierte DWST-5 Protein zeigt eine hohe Spezifität für *Salmonella* spp. und verfügt überdies über vorhergesagte 14 antigene Epitope und acht Oberflächen-exponierte Bereiche in seiner Sequenz (Figur 9).

Das *ompD* Gen wurde mittels PCR von chromosomaler DNA des SALMOPORC^{®} Stammes amplifiziert und "in frame" in den Expressionsvektor pGEX5x-3 kloniert. Mit dem so konstruierten Plasmid pSOM500 konnte in *E. coli* ein rekombinantes Glutathion S-Transferase-OmpD-Fusionsprotein (GST-OmpD) hergestellt werden.

Die Entwicklung des Negativ-Lebendmarker-Impfstoffes ist an die Entwicklung eines ELISAbasierten Diagnosesystems gekoppelt, welches in der Praxis die Unterscheidung von geimpften und erkrankten Tieren ermöglicht. Hierzu wurde das rekombinante GST-OmpD Protein als Festphasenantigen auf Polysorb^{®} ELISA-Platten aufgebracht und mit verschiedenen, bereits mit einem kommerziellen ELISA System zur S. Typhimurium Diagnostik eingesetzten Seren getestet.

Ein Nullserum aus einem Impfversuch mit dem verwendeten Impfstoff SALMOPORC^{®} diente als Negativ-Kontrolle, ein Hyperimmun-Serum nach der vierten Immunisierung als Positiv-Kontrolle. Die Versuche zeigen, dass alle im kommerziellen ELISA positiven Seren im OmpD-ELISA eine mindestens doppelt so hohe Absorption wie die Negativkontrolle aufwiesen.

Bei einem Massenscreening mit Schweineseren bei dem parallel mit dem kommerziellen Salmotype Pigscreen^{®} ELISA untersucht wurde, kamen Platten mit 0,5 µg/ml GST-OmpD-Aggregatpräparation und Platten mit 0,5 µg/ml säulenchromatographisch aufgereinigtem Fusionsprotein zum Einsatz. Beide reagierten sehr unterschiedlich, wobei davon auszugehen ist, dass im Falle der nicht aufgereinigten Aggregatpräparation vermehrt Kreuzreaktionen mit *E. coli* Proteinen auftraten. Im ELISA mit dem aufgereinigten Fusionsprotein, reagieren mehr Herden positiv als im Salmotype Pigscreen ELISA, was entweder für eine höhere Sensitivität, oder aber für eine geringere Spezifität spricht.

### 4.2. Konstruktion eines isogenen Lebend-Negativmarker-Impfstoffes mit der Deletion des ompD Gens in S. Typhimurium

Für die Mutagenese des *ompD* Gens in *S*. Typhimurium wurde mit pROKB1 ein Vektor gewählt, der über ein R6K Replikon verfügt. Da dieses Replikon für die Replikation im Bakterium zwingend das Produkt des Lambda-Phagen-Gens *pir* benötigt, kann der Vektor in *Salmonella* als "suicide" Vector eingesetzt werden.

Ein verkürztes *ompD-Gen* wurde aus zwei PCR-Fragmenten hergestellt, die von Bereichen am 5'- und am 3'-Ende des Gens amplifiziert wurden. Die zur Gen-Mitte hin lokalisierten Primer tragen jeweils eine BsmBI Restriktionsenzymschnittstelle (McCaffery et al., 1996, A novel system for the rapid generation of precise DNA deletions. Nucleic Acids Res. 24, 5048-5050). Beide Stücke wurden im Anschluss mit BsmBI geschnitten und ligiert. Dadurch entstand eine "in frame" Mutation im Gen, die keinerlei Fremd-DNA enthält. Die ligierten Einzelfragmente wurden dann zunächst in den Vektor pTOPO 2.1 (pSOM800) kloniert und dann in den Vektor pROKB1 (pSOM14666) umgesetzt.

Das Mutagenisierungsplasmid pSOM14666 sollte durch Konjugation von dem DAPI-auxotrophen *E.coli* Stamm β2155 als Plasmid tragendem Donor auf den S. Typhimurium Impfstamm Salmoporc^{®} als Rezipienten übertragen werden. Nach Selektion (auf Kanamycinhaltigen Platten) und Gegenselektion (in Saccharose-haltigem Medium) sollten durch PCR Screening Klone isoliert werden, bei denen eine homologe Rekombination stattgefunden hat. Das Protokoll für die Konjugation wurde im Vorfeld für *Actinobacillus pleuropneumoniae* etabliert und musste für S. Typhimurium angepasst werden, da in den ersten Experimenten mit der Standardmethode keine stabilen Deletionsmutanten erzeugt werden konnten. Hierbei wurde das Anzuchtsmedium (Blutagar), sowie die Anzuchtszeit des Salmonellen Rezipienten optimiert.

Nach Optimierung der Konjugationsmethode (Anzucht des Rezipienten auf Blutagar über ca. 40 Stunden bei 37°C) konnten Salmonellen Transkonjugaten mit integriertem Mutagenisierungsplasmid isoliert werden ("single crossing-overs"). Mit diesen Isolaten wurde eine Gegenselektion mit Saccharose durchgeführt. Hierbei wird auf die Erreger selektiert, die über einen zweiten Rekombinationsschritt das Plasmid aus ihrem Genom entfernt haben (diese Erreger tragen nicht mehr das Gen für die Levansucrase und sind somit nicht Saccharose empfindlich). Auch hier mussten die Anzuchtsbedingungen im Vergleich zum etablierten Protokoll angepasst werden; so war es erforderlich, die Erreger nach Anzucht im Gegenselektionsmedium für 72 Stunden bei 4°C zu halten, um eine gute Ausbeute an "double-crossing-overs" zu erhalten. Klone aus der Gegenselektion wurden im Anschluss in einer PCR auf ihren *ompD* Genotyp überprüft, um sicher zu stellen, dass sie das verkürzte *ompD* Gen tragen.

Auf diese Weise konnte eine isogene *ompD* Knockout Mutante erstellt werden, die über PCR und Southern-Blot überprüft wurde. Wie in Figur 10 gezeigt, wurde ein Enzym (Pstl) gewählt, dass bei Wildtyp und Mutante je vor und hinter dem Zielgen schneidet und beim Wildtyp ein ca. 3900 bp großes Fragment, bei der Mutante ein ca. 2900 bp großes Fragment ergibt. Des Weiteren wurde ein Enzym (BspHI) gewählt, dass innerhalb des Wildtypgens schneidet, nicht aber im verkürzten Gen der Mutante, wobei sich für den Wildtyp Fragmente der Größe 6000 bp und 5000 bp ergeben, für die Mutante ein Fragment von ca. 9300bp.

## Patentansprüche

1. Bakterieller Impfstoff für die Anwendung bei einem Tier, insbesondere einem Nutztier,
umfassend
mindestens einen Serotyp des Organismus *Salmonella enterica* mit mindestens jeweils einer Knock-out Mutation in mindestens drei verschiedenen chromosomalen Genen, wobei der mindestens eine Serotyp *Salmonella* Typhimurium mit mindestens einer Knock-out Mutation in einem Gen kodierend für das Protein OmpD umfasst.

2. Impfstoff nach Anspruch 1 **dadurch gekennzeichnet, dass** die mindestens jeweils eine Knock-out Mutation eine unmarkierte Deletion ist.

3. Verfahren zur Herstellung eines Impfstoffes nach einem der Ansprüche 1 oder 2, **dadurch gekennzeichnet, dass** zur Mutagenese von OmpD im Serotyp *Salmonella* Typhimurium ein pROKB1 Vektor mit einem R6K Replikon verwendet wird, welcher das OmpD Gen mit einer Knock-out Mutation enthält.

4. Bakterium verwendet in einem bakteriellen Impfstoff nach mindestens einem der Ansprüche 1 bis 2.

5. Lebend-Negativmarker-Impstoff **umfassend** mindestens einen bakteriellen Impfstoff nach mindestens einem der Ansprüche 1 bis 2.

6. Inaktivat-Negativmarker-Impstoff **umfassend** mindestens einen bakterieller Impfstoff nach mindestens einem der Ansprüche 1 bis 2.

7. Immunogenes Protein synthetisiert von mindestens einem bakteriellen Infektionserreger eines Tieres in einem Impfstoff nach mindestens einem der Ansprüche 1 bis 2, **dadurch gekennzeichnet, dass** es Antikörper generiert, die bevorzugt für eine protektive Immunantwort nicht essentiell sind, und wobei es mit Antikörpern aus mindestens einem Serum von mit *Salmonella* Typhimurium infizierten Schweinen und mit Antikörpern aus mindestens einem Serum von einem mit einem Impstoff gegen *Salmonella* Typhimurium geimpften Schweinen wechselwirkt, wobei das immunogene Protein das Protein OmpD aus *Salmonella* Typhimurium umfasst.

8. Immunogenes Protein nach Anspruch 7 , **dadurch gekennzeichnet, dass** es in Form eines Glutathion S-Transferase-OmpD Fusionsproteines vorliegt.

9. Verwendung eines immunogenen Proteins nach einem der Ansprüche 7 oder 8 zur Detektion von natürlich infizierten, von mit einem bakteriellen Impfstoff nach mindestens einem der Ansprüche 1 bis 2 geimpften Tieren und / oder von natürlich infizierten und mit einem bakteriellen Impfstoff nach mindestens einem der Ansprüche 1 bis 2 geimpften Tieren, insbesondere Schweinen.

10. Diagnostisches Verfahren zur Unterscheidung von infizierten Tieren, von mit einem bakteriellen Impfstoff nach mindestens einem der Ansprüche 1 bis 2 geimpften Tieren und / oder von natürlich infizierten und mit einem bakteriellen Impfstoff nach mindestens einem der Ansprüche 1 bis 2 geimpften Tieren umfassend die serologische Bestimmung von für die protektive Immunantwort nicht essentiellen immunogenen Proteinen, insbesondere OmpD, in natürlich infizierten Tieren und/oder in mit einem bakteriellen Impfstoff nach mindestens einem der Ansprüche 1 bis 2 geimpften Tieren.

11. Diagnostisches Verfahren nach Anspruch 10, **dadurch gekennzeichnet, dass** es in Form eines ELISA-Tests durchgeführt wird.

12. Screening-Platten, insbesondere ELISA-Platten, mit einem immunogenen Protein nach einem der Ansprüche 7 oder 8.

## Claims

1. A bacterial vaccine for use in an animal, in particular a domestic animal,
comprising
at least one serotype of the organism *Salmonella enterica* with at least one knock-out mutation each in at least three different chromosomal genes, wherein the at least one serotype comprises *Salmonella* Typhimurium with at least one knock-out mutation in a gene coding for the protein OmpD.

2. The vaccine according to claim 1, **characterized in that** the at least one knock-out mutation each is an unmarked deletion.

3. A method for producing a vaccine according to any of claims 1 or 2, **characterized in that** for the mutagenesis of OmpD in the serotype *Salmonella* Typhimurium a pROKB1 vector with an R6K replicon is used, which contains the OmpD gene with a knock-out mutation.

4. A bacterium used in a bacterial vaccine according to at least one of claims 1 to 2.

5. A conventional negative-marker vaccine, comprising at least one bacterial vaccine according to at least one of claims 1 to 2.

6. An inactivated negative-marker vaccine, comprising at least one bacterial vaccine according to at least one of claims 1 to 2.

7. An immunogenic protein synthesized by at least one bacterial infective agent of an animal in a vaccine according to at least one of claims 1 to 2, **characterized in that** it generates antibodies which preferably are not essential for a protective immune response, and wherein it interacts with antibodies from at least one serum of pigs infected with *Salmonella* Typhimurium and with antibodies from at least one serum of pigs vaccinated with a vaccine against *Salmonella* Typhimurium, wherein the immunogenic protein comprises the protein OmpD from *Salmonella* Typhimurium.

8. The immunogenic protein according to claim 7, **characterized in that** it is present in the form of a glutathione S-transferase-OmpD fusion protein.

9. Use of an immunogenic protein according to any of claims 7 or 8 for the detection of naturally infected animals, animals vaccinated with a bacterial vaccine according to at least one of claims 1 to 2 and/or of naturally infected animals, in particular pigs, vaccinated with a bacterial vaccine according to at least one of claims 1 to 2.

10. A diagnostic method for distinguishing infected animals, animals vaccinated with a bacterial vaccine according to at least one of claims 1 to 2 and/or naturally infected animals vaccinated with a bacterial vaccine according to at least one of claims 1 to 2, comprising the serological determination of immunogenic proteins, in particular OmpD, not essential for the protective immune response in naturally infected animals and/or in animals vaccinated with a bacterial vaccine according to at least one of claims 1 to 2.

11. The diagnostic method according to claim 10, **characterized in that** it is carried out in the form of an ELISA test.

12. Screening plates, in particular ELISA plates, with an immunogenic protein according to any of claims 7 or 8.

## Revendications

1. Vaccin bactérien pour son utilisation chez un animal, en particulier, un animal d'élevage,
comprenant
au moins un sérotype de l'organisme *Salmonella enterica* comportant au moins respectivement une mutation knock-out dans au moins trois gènes chromosomiques différents, dans lequel l'au moins un sérotype comprend *Salmonella* Typhimurium comportant au moins une mutation knock-out dans un gène codant pour la protéine OmpD.

2. Vaccin selon la revendication 1, **caractérisé en ce que** l'au moins respectivement une mutation knock-out est une délétion non marquée.

3. Procédé de production d'un vaccin selon l'une des revendications 1 ou 2, **caractérisé en ce que**, pour la mutagenèse de l'OmpD dans le sérotype de *Salmonella* Typhimurium, on utilise un vecteur pROKB1 avec un réplicon R6K qui contient le gène OmpD comportant une mutation knock-out.

4. Bactérie utilisée dans un vaccin bactérien selon au moins l'une des revendications 1 à 2.

5. Vaccin vivant à marqueur négatif comprenant au moins un vaccin bactérien selon au moins l'une des revendications 1 à 2.

6. Vaccin inactivé à marqueur négatif comprenant au moins un vaccin bactérien selon au moins l'une des revendications 1 à 2.

7. Protéine immunogène synthétisée à partir d'au moins un agent infectieux bactérien d'un animal dans un vaccin selon au moins l'une des revendications 1 à 2, **caractérisée en ce qu'**elle génère des anticorps qui, de préférence, ne sont pas essentiels pour une réponse immunitaire protectrice, et dans lequel elle interagit avec des anticorps provenant d'au moins un sérum des porcs infectés avec *Salmonella* Typhimurium et avec des anticorps provenant d'au moins un sérum des porcs vaccinés avec un vaccin contre *Salmonella* Typhimurium, la protéine immunogène comprenant la protéine OmpD de *Salmonella* Typhimurium.

8. Protéine immunogène selon la revendication 7, **caractérisée en ce qu'**elle se présente sous la forme d'une protéine de fusion glutathion S-transférase-OmpD.

9. Utilisation d'une protéine immunogène selon l'une des revendications 7 ou 8, pour la détection d'animaux infectés naturellement, d'animaux vaccinés par un vaccin bactérien selon au moins l'une des revendications 1 à 2, et/ou d'animaux infectés naturellement et vaccinés avec un vaccin bactérien selon au moins l'une des revendications 1 à 2, en particulier des porcs.

10. Procédé diagnostique pour distinguer des animaux infectés, d'animaux vaccinés par un vaccin bactérien selon au moins l'une des revendications 1 à 2 et/ou d'animaux infectés naturellement et vaccinés par un vaccin bactérien selon au moins l'une des revendications 1 à 2, comprenant la détermination sérologique de protéines immunogènes non essentielles pour la réponse immunitaire protectrice, en particulier, OmpD, chez des animaux infectés naturellement et/ou des animaux vaccinés avec un vaccin bactérien selon au moins l'une des revendications 1 à 2.

11. Procédé diagnostique selon la revendication 10, **caractérisé en ce qu'**il est réalisé sous la forme d'un test ELISA.

12. Plaques de criblage, en particulier, plaques pour ELISA, comportant une protéine immunogène selon l'une des revendications 7 ou 8.


### SEQUENCE LISTING

<110> Gerlach, Prof. Gerald F
<120> Bakterieller Impfstoff
<130> GRL104
<160> 2
<170> PatentIn version 3.1
<210> 1
   <211> 1272
   <212> DNA
   <213> Mycoplasma hyopneumoniae
<220>
   <221> Mhp378 C_region
   <222> (1)..(1272)
   <223> carboxyterminus von Mhp378 beginnend an Nukleinsäureposition 856 bis 2127
<400> 1
   attcttcgtt cttttccaga ggccctttca ggaggatcca ctgattcggc aaaatcagtt     60
   ttaggaattg ataatcaagc aacgctagtt tttgctcttg ccagatcagt ttctgaaggt    120
   aatcgatccc aggaagttac cgttcttgat aggcaaaaga atttaattga ttatatatct    180
   tttatagata aacctgattc aattaggtat aaaaatttag aaaaaatttt taatttatta    240
   agccaaggga taaaagatcg ctcaatttat tatacatctg caggggagta taattcaact    300
   tttttccgga atcatcagca ggttttctca attggttcaa cttcaggcta tttccataat    360
   tttgttaaac caacagcgac aaattatcaa atcggcttta agaaaaatga tggtcttaag    420
   tcagtttata gcgttagcta ccccaaattt agcgcaattg tatcacttga agatctcaag    480
   gatataacca aagatctaga aataacagca accgatggta gctctaaatt aaaaattgat    540
   gctaaatttt taggaaaact taaagaatat gcacagcaaa atccagttaa aaaagtgttt    600
   tattttactg atcgatcaga aaaaccttca ggtatcttcg aaaaagatta tattgtttta    660
   ggcaaataca aaaatgacaa aaacgaagaa tttaatggcc ttgtaattcc aacttataca    720
   gaactctata aaaattctgg atcaaatgcc cttaatgatg atgaacttgc acttgaagcc    780
   ccaccgcata aattcgatgc aaatagtaaa atcaccccca ttgtcgccca aggtcctgat    840
   ctaattttta ttcattcaac tgaaaaagaa gataaagccg caaaagcttt tgttaaatgg    900
   cttttgacag aaaaaatagt ctttgaggaa aatagtcagg aaaaaatgac tccgcttgag    960
   tattttgcca gagcaacttc atatttatta ccaataaaat caacacttga taaaacccat   1020
   tttagtccaa aaaatagatc acagaaattt atacttgacc aatttagtaa atttcttaat   1080
   gctgattcaa aaggaaaata ttcgcttgtc tatgataatg ccgatgcaaa cgcttcatcc   1140
   ttccgtgaat cactagattc ttcagttgcc cagatgcaat cattaaaagc cagcgatgga   1200
   aaagtgcgta gttttaaaga gtttttagaa aaactagagg gaaatttagg tcctgctttt   1260
   aaatcaaaat aa                                                        1272
<210> 2
   <211> 1095
   <212> DNA
   <213> Mycoplasma hyopneumoniae
<220>
   <221> Mhp651 C_region
   <222> (1)..(1095)
   <223> Carboxyterminus von Mhp651 beginnend an Nukleinsäureposition 1051 bis 2142
<400> 2
   tggacttcag attatcttgc agaagaaacc ggaaatggcc ttgatttagt catcgatggg   60
   cattcgcata caaaaatcga aattcacaaa cccaaagaag ataaaaaagt ttgggtaacc  120
   caaactgagg cctatgcaaa atggctcggg gacattgatt tagtttttga tactgaaact  180
   ggcgagatag taaaaattgt acaatcttta agagatatta accaaatcaa tattgtaacc  240
   agagatttat cagaacatta tatcaaaaaa cttcataaag tttatgatgt agaaaatgat  300
   gtaaaagtat ttaattcgcc tggtgttttt gaacatgttc aatcaattga gatcaacaaa  360
   actccttact ggattggacg ggtaaaacca acttcattag gggtaatgac agcagatgcc   420
   attgcttggg aatatgcaaa aagttcaaaa gaacaagtac aaagtacgaa aaatgaaatt  480
   gcaacacttg ataattcctt aggacttata aacggtggta gtcttagaac agatctaaaa  540
   agtggtgaaa tcaaaagagg agatgtttta ggggttagtc cttttgggaa taggattgta  600
   actattaaac taaaaggaga tacacttaaa aaaactctcg aatacggact ttctatgggg  660
   aaacaaggcg catttgccca actatcttca aatatttcct ataaagtcaa agttgaaaaa  720
   gggactgatc cgaaaaccaa aatagaatcc tgggtttgga aaccagatac aacatccttt  780
   aaaattaata acaagccgat tgatgataac aaattttact atttaagcac aaatgattat  840
   ctttcagcgg gcggggacgg ctatcaaatg ttaaatctag gtaaaaacag ggatattgaa  900
   aaagtttatg agggagttca atatattgat tctttaatta aatatggtca atatttggac  960
   aaattaacta aagattcaag tcaaaaagat ctatttgccc acacattcca agaatattta 1020
   agttctgatt ttacaaaaaa tcaacaagtt gagattcctc aagaagctct aacgaaaagc 1080
   caaagtcaat cttaa                                                  1095